# EUROPÄISCHE PATENTANMELDUNG

(11) **EP 3 456 356 A1**
(43) Veröffentlichungstag der Anmeldung: **20.03.2019**
(21) Anmeldenummer: 18180512.8
(22) Anmeldetag: 21.12.2010
(51) Int. Cl.: A61K 47/54, A61K 47/59, A61K 47/64, A61K 48/00, A61P 11/00, C12N 15/88

(54) **KONJUGAT MIT ZIELFINDUNGSLIGAND UND DESSEN VERWENDUNG**

(30) Priorität: 21.12.2009 EP 09015812
(62) Teilanmeldung aus: 10805775.3
(71) Anmelder: ethris GmbH, 82152 Planegg (DE)
(72) Erfinder: RUDOLPH, Carsten, 82152 Krailing (DE); GEIGER, Johannes-Peter, 81249 München (DE)
(74) Vertreter: Vossius & Partner Patentanwälte Rechtsanwälte mbB

(57) **Zusammenfassung**

Es wird ein Konjugat aus Agens-Komplex und mindestens einem Zielfindungsliganden, wobei der Agens-Komplex ein Agens umhüllt von einem Hüllmaterial umfasst und wobei der Zielfindungsligand ein Prostacyclinanalogon ist, sowie dessen Verwendung beschrieben

## Beschreibung

Die Erfindung betrifft ein Agens enthaltende Konjugate, die als Zielfindungsstruktur ein Prostacyclinanalogon aufweisen und die Verwendung solcher Konjugate für die Gentherapie bzw. den Gentransfer in Bronchial- und Alveolarepithelzellen,

Die Lunge ist einerseits ein Organ, dessen Funktion lebensnotwendig ist und andererseits ist die Lunge ein Organ, das aufgrund seiner großen Oberfläche und seiner Zugänglichkeit attraktiv ist, um Wirkstoffe oder aktive Mittel in den Körper einzuschleusen.

Es ist schon lange bekannt, über Aerosole, Vernebler, Inhalatoren oder Pumpsprays aktive Mittel in die Lunge zu bringen sowohl für lokale als auch systemische Wirkung. Bekannt ist es auch, zur Gentherapie virale oder nicht virale Gentransfermittel über die Lunge zu verabreichen. Sowohl virale als auch nicht virale Trägermittel sind vielfach mit Nebenwirkungen bei ihrer Anwendung verbunden. Dies ist insbesondere dadurch bedingt, dass die Dosis relativ hoch sein muss, da der Gentransfer, d.h. das Einschleusen der gewünschten Gene in Zellen häufig nicht effizient genug ist. Es wurde daher schon lange nach Mitteln gesucht, um die Effizienz des Gentransfers zu verbessern. Hierzu wurde bereits vorgeschlagen, Gene mit einem kationischen Lipid zu umhüllen, da kationische Teilchen leichter phagozytiert werden können. Ein hierzu vorgeschlagenes Mittel, das bereits in klinischen Versuchen getestet wird [7], ist Genzyme Lipid 67. Bekannt ist auch die Verwendung von Polyethyleniminpolymeren (PEI), um Nukleinsäuren zu umhüllen [8]. Obwohl PEI DNA schützen kann, ist es mit dem Nachteil verbunden, dass die Gentransfereffizienz gering ist, außerdem wurde gefunden, dass es in hoher Dosis, die aufgrund der geringen Transfektionseffizienz erforderlich ist, Entzündungen hervorruft.

Es wurde daher auch schon versucht, mit kationischen Polymeren umhüllte Teilchen mit Liganden zu versehen, die die Teilchen in Zellen einschleusen sollen. Versuche gab es bereits mit Transferin [10], Folsäure [11], Lactoferin [12], Clenbuterol [13] und Wachstumsfaktoren, wie EGF [14]. Obwohl mit diesen Liganden der PEI-vermittelte Gentransfer verbessert werden konnte, besteht immer noch Bedarf dafür, aktive Mittel der Lunge gezielt und in hoher Ausbeute zuzuführen.

Weiterhin wurde und wird versucht, neue Wege für die therapeutische Behandlung von chronischen Lungenkrankheiten zu finden, für die der Gentransfer ein viel versprechender Ansatz ist. Auf angeborenen oder erworbenen Protein- und/oder Gendefekten beruhende Lungenkrankheiten könnten durch Bereitstellung der fehlenden oder geschädigten Proteine oder Genprodukte verbessert, gelindert oder sogar geheilt werden. Dazu muss jedoch die Verabreichung regelmäßig erfolgen. Ein Ausgleich zwischen unerwünschten Nebenwirkungen und erwünschter therapeutischer Wirkung muss daher gefunden werden. Außerdem ist auch die erforderliche Dosierungshäufigkeit für eine länger andauernde Therapie ein wichtiger Aspekt.

Aufgabe der Erfindung war es daher, Konjugate bereitzustellen, die es ermöglichen, Wirkstoffe oder aktive Mittel, die zur Behandlung oder Linderung von Lungenkrankheiten geeignet sind, in einer Form bereitzustellen, die von Zellen in der Lunge gezielt aufgenommen werden können, insbesondere von Bronchial- und Alveolarepithelzellen.

Diese Aufgabe wird gelöst mit einem Konjugat, wie es in Anspruch 1 definiert ist.

Überraschenderweise wurde gefunden, dass Epithelzellen in der Lunge, d.h. Bronchialepithelzellen und Alveolarepithelzellen IP₁-Rezeptoren tragen und dass diese Rezeptoren für einen effizienten Transfer von wirkstoffhaltigen Teilchen adressiert werden können. Mit den erfindungsgemäßen Konjugaten gelingt es, Epithelzellen in den Bronchien und Alveolen gezielt über diese IP₁-Rezeptoren anzusteuern durch Verwendung mindestens eines Prostacyclinanalogons als Zielfindungsstruktur.

Im Folgenden wird der Gegenstand der Erfindung detailliert beschrieben und die Eigenschaften und Vorteile werden näher ausgeführt. Die Erfindung wird auch in den beigefügten Figuren näher erläutert, wobei
Fig. 1 die Ergebnisse einer Western-Blot-Analyse der Expression von IP₁-Rezeptor in menschlichen Alveolar- und Bronchialepithelzellen zeigt.
Fig. 2 die Fluoreszenzintensität von A549- und 16HBE14o-Zellen nach Inkubation mit FLUO-BSA-ILO bzw. FLUO-BSA-TRP zeigt.
Fig. 3a die Fluoreszenzintensität von FLUO-BSA im Vergleich zu FLUO-BSA-ILO in verschiedenen Zelllinien zeigt; Fig. 3b und Fig. 3c die mittleren Fluoreszenzintensitäten zeigen bei steigenden Konzentrationen von CAY10449 bzw. ILO; Fig. 3d die mittlere Fluoreszenzintensität von FLUO-BSA-ILO nach Zugabe von CAY10449 zeigt; Fig. 3e Aufnahmen einer konfokalen Laserrastermikroskopie zur Oberflächenbindung zeigt.
Fig. 4 die DNA-Freisetzung für PEI-g-ILO-Konstrukte mit unterschiedlichen Verhältnissen von N/P zeigt.
Fig. 5a den Expressionsgrad für mit PEI-g-ILO-Genvektoren transfizierte Zellen im Vergleich zu unmodifiziertem PEI zeigt; Fig. 5b die Genexpression für PEI-g-ILO im Vergleich zu PEI zeigt; Fig. 5c die Expressionsgrade für PEI-g-ILO in A549- und BEAS-2B-Zellen im Vergleich zu PEI zeigt.
Fig. 6a in vivo Studien zur Genexpression von Luciferase zeigt; Fig. 6b die Luciferaseexpression in homogenisiertem Lungengewebe, das von Mäusen erhalten wurde, die PEI-g-ILO F_{ILO} = 5 Genvektoren erhalten hatten, im Vergleich zu PEI-Genvektoren zeigt.
Fig. 7a die metabolische Aktivität von unbehandelten Zellen im Vergleich zu mit PEI oder erfindungsgemäßem Konstrukt behandelten Zellen zeigt; Fig. 7b die Veränderung des Cytokinspiegels nach Verabreichung von PEI oder erfindungsgemäßem Konstrukt im Vergleich zu unbehandelten Zellen zeigt.
Fig. 8 die dosisabhängige Genvektorzuführung in Lungenzellen zeigt.

Es wurde überraschenderweise gefunden, dass Konjugate, die Prostacyclinanaloga als Zielfindungsstruktur tragen für die Adressierung von Epithelzellen in der Lunge, insbesondere von Bronchial- und Alveolarzellen geeignet sind und mit hoher Effizienz Agentien in die Zellen schleusen können. Damit wird nun erfindungsgemäß ein Mittel bereitgestellt, um aktive Inhaltsstoffe in Lungenepithelzellen einzuschleusen. Dies bietet neue Möglichkeiten der therapeutischen Behandlung von unterschiedlichsten Lungenkrankheiten.

Prostacyclin gehört zur Klasse der Prostaglandine und ist als Prostaglandin I₂ bzw. PGI₂ bekannt; es adressiert und bindet den Prostacyclin-(IP₁)-Rezeptor. Der IP₁-Rezeptor ist ein 7-Transmembran-G-proteingekoppelter Rezeptor, der vor allem auf Endothelzellen gefunden wurde, insbesondere auf Muskelzellen z.B. auf Muskelzellen von Blutgefäßen [15-17]. Die Bindung von Prostacyclin an einen IP₁-Rezeptoragonisten führt zu einer endosomalen Internalisierung von Rezeptor/Ligandenkomplexen über Clathrin-vermittelte Prozesse [18, 19]. Die Erfinder der vorliegenden Erfindung haben nun gefunden, dass diese Wirkung dazu genutzt werden kann, den gezielten Transfer von aktiven Mitteln in Alveolar- und Bronchialepithelzellen zu verbessern und die Aufnahme von für die Lunge nützlichen oder Lungenleiden therapierenden aktiven Mittel zu ermöglichen.

Erfindungsgemäß wird daher ein Konjugat bereitgestellt, das als Zielführungsstruktur für Bronchien- und Alveolarepithelzellen mindestens ein Prostacyclinanalogon aufweist. Prostacyclin selbst ist zu instabil und wird zu schnell abgebaut, um für den vorgesehenen Zweck verwendet werden zu können. Es sind jedoch stabile Prostacyclinanaloga bekannt, die ebenfalls an den IP₁-Rezeptor binden und als Agonisten wirken. Als Prostacyclinanalogon wird hier eine Verbindung bezeichnet, die von Prostansäure abgeleitet ist, die eine dem Prostacyclin vergleichbare oder höhere Bindungsfähigkeit an den IP₁-Rezeptor hat und eine höhere Stabilität als natürliches Prostacyclin aufweist. Derartige Verbindungen sind bekannt. Für die erfindungsgemäßen Konjugate sind die bekannten Prostacyclinanaloga geeignet.

Bevorzugt werden als Liganden Iloprost und/oder Treprostinil, zwei als Arzneimittel zugelassene Pröstacyclinanaloga verwendet. Andere Prostacyclinanaloga können ebenfalls verwendet werden.

Ein für die Erfindung geeignetes Prostacyclinanalogon ist ein solches, das in physiologischer Umgebung und/oder bei der Lagerung stabiler ist als das natürlich vorkommende Prostacyclin. Wie oben ausgeführt wird Prostacyclin sehr schnell abgebaut, es hat in physiologischer Umgebung, d.h. im Blut, eine Halbwertszeit von nur wenigen Minuten und kann nicht lange aufbewahrt werden. Erfindungsgemäß geeignet sind daher solche Prostacyclinanaloga, die in physiologischer Umgebung mindestens 20 Minuten, bevorzugt mindestens 30 Minuten, noch bevorzugter mindestens 45 Minuten ihre Eigenschaften behalten, ohne abgebaut oder inaktiviert zu werden bzw. solche die in physiologischer Umgebung eine Halbwertszeit von mindestens 15 Minuten, bevorzugt mindestens 20 Minuten, bevorzugter mindestens 25 Minuten haben. Unter Halbwertszeit wird hier allgemein die Zeitspanne verstanden, in der in physiologischer Umgebung die Hälfte des Ausgangsmaterials - hier das Prostacyclinanalogon, zerfallen ist bzw. inaktiviert oder umgewandelt wurde. Die Halbwertszeit kann einfach in üblicher Weise festgestellt werden, z.B. indem das inbetracht kommende Prostacyclinanalogon in eine physiologische Lösung bei einer Temperatur von 35-37°C gegeben wird und der Gehalt an unzersetztem Prostacyclin zu Anfang und nach vorbestimmten Zeiträumen bestimmt wird.

Ein für die Erfindung geeignetes Prostacyclinanalogon ist weiterhin ein solches, das eine mit Prostacyclin vergleichbare oder höhere Bindungsfähigkeit an den IP₁-Rezeptor hat. Ein Verfahren, um die Bindungsfähigkeit eines Prostacyclinanalogons festzustellen, ist ein kompetitives Verfahren, bei dem Prostacyclin oder ein bekanntes Prostacyclinanalogon wie Iloprost und/oder Treprostinil sowie ein Prostacyclinanalogönkandidat mit Fluorescein und Rinderserumalbumin (BSA) konjugiert werden und dann zu verschiedenen Lungenzelllinien zugegeben werden, wobei dann die Bindung und zelluläre Aufnahme durch Durchflusscytometrie und konfokale Laserrastermikroskopie untersucht werden. Ein Kandidat, der genauso stark oder stärker wie Prostacyclin oder Iloprost bzw. Treprostinil bindet, ist als Teil des erfindungsgemäßen Konjugats ebenfalls bevorzugt. Gleiche Bindefähigkeit bedeutet, wenn der fluoresceinniarkierte Kandidat mindestens im selben Ausmaß wie fluoresceinmarkiertes Prostacyclin oder fluoresceinmarkiertes Iloprost bzw. fluoresceinmarkiertes Treprostinil bindet. Ist der Anteil an fluoresceinmarkierten Kandidaten niedriger, bedeutet dies, dass Prostacyclin oder Iloprost bzw. Treprostinil den Kandidaten aus der Bindung drängen, so dass dessen Bindefähigkeit nicht so hoch ist.

Die bekannten Prostacyclinanaloga werden verwendet zur Behandlung von pulmonalem arteriellem Hochdruck (PAH) und werden üblicherweise intravenös oder als Aerosol verabreicht [20], sie müssen häufig über den Tag verteilt dosiert werden, um diesen Zweck zu erfüllen. Für die vorliegende Erfindung wird das Prostacyclinanalogon jedoch als Zielfindungsstruktur eingesetzt und dient nicht zur Behandlung des pulmonalen arteriellen Hochdrucks. Es wurde gefunden, dass in der erfindungsgemäßen Kombination mit kationischer Hüllsubstanz und Agens Prostacyclinanaloga eine entzündungshemmende Wirkung aufweisen und hierdurch die Wirkung der erfindungsgemäßen Konjugate noch verbessern.

Der zweite Teil des erfindungsgemäßen Konjugats ist ein Agenskomplex, d.h. ein von einem Hüllmaterial umhülltes Agens. Das Hüllmaterial dient dazu, das Agens zu schützen und gleichzeitig die Aufnahme in die Zelle nicht zu stören oder ggf. sogar zu verbessern.

Der als wirkendes Prinzip oder Wirkstoff aktive Bestandteil des erfindungsgemäßen Konjugats kann jedes Agens außer Prostacyclin sein, das in den von dem erfindungsgemäßen Zielfindungsliganden adressierten Zellen eine vorteilhafte, heilsame, lindernde oder modulatorische Wirkung ausübt. Als Beispiele für erfindungsgemäß verwendete Agentien sind insbesondere Nukleinsäuren, Peptide oder Polypeptide, Wirkstoffe oder Tracer und/oder Derivate der genannten Substanzen und/öder Mischungen davon. Das erfindungsgemäß verwendete Agens soll bevorzugt ein Mittel sein, dass ein Lungenleiden lindern oder heilen kann. Erfindungsgemäß wird ein Prostacyclinanalogon hier nicht als Wirkstoff sondern nur als Zielfindungsligand verwendet.

Gemäß einem Aspekt der vorliegenden Erfindung ist das Agens eine Nukleinsäure, die ein Gen oder Genfragment umfasst, dessen Defekt oder Mangel eine Lungenkrankheit hervorruft oder die ein immunmodulierend wirksames Protein, insbesondere ein Antigen kodiert. Die Nukleinsäure kann eine DNA oder RNA sein und sie kann ein oder mehrere Gene oder Fragmente aufweisen. Die Nukleinsäure kann eine autonom replizierende oder integrierende Sequenz sein, sie kann als Plasmid, Vektor oder in anderer dem Fachmann wohlbekannten Form sein. Sie kann linear oder ringförmig und einzel- oder doppelsträngig sein. Jede in einer Zelle aktive Nukleinsäure ist hier geeignet. Außerdem kann die Nukleinsäure in an sich bekannter Weise weitere Elemente, die für die Expression des Gens notwendig oder nützlich sind, z.B. Promotoren, Enhancer, Signalsequenzen etc. aufweisen.

In einer weiteren Ausführungsform ist der aktive Bestandteil des erfindungsgemäßen Konjugats ein Peptid, Polypeptid, Protein oder Proteinfragment, das geeignet ist, einen Proteinmangel oder Proteindefekt, der zu einer Lungenkrankheit führt, zu beheben oder das immunmodulatorisch wirkt.

Weiterhin kann der aktive Bestandteil des erfindungsgemäßen Konjugats ein Wirkstoff sein, der, wenn er in einer Bronchial- und/oder Alveolarepithelzelle vorhanden ist, zur Heilung oder Linderung eines krankhaften Zustands in der Lunge führt. Beispiele hierfür sind z.B. entzündungshemmende Mittel wie Steroide, die zur Behandlung von Asthma eingesetzt werden. Da auch der Ligand entzündungshemmend wirkt, führt diese Kombination zu einem sehr wirkungsvollen Mittel.

In einer weiteren Ausführungsform kann das Agens ein Reportermolekül sein, dessen Aufnahme in die Zelle diagnostisch wichtige Informationen liefern kann. Reportermoleküle, die für die Diagnostik geeignet sind, sind dem Fachmann bekannt und Beispiele für geeignete Reportermoleküle sind radioaktive oder fluoreszierende Tracermoleküle, die dem Fachmann bekannt sind. Die Reportermoleküle können z.B. eingesetzt werden, um den Verlauf einer Behandlung oder den Zustand der Lunge zu überprüfen.

Ein weiterer wesentlicher Bestandteil des erfindungsgemäßen Konjugats ist ein Hüllmaterial, das das Agens umhüllt, um es vor Abbau oder Veränderung zu schützen und das die Einschleusung in die Zelle nicht stört oder sogar fördert. Das Hüllmaterial ist geeigneterweise ein kationisches oder neutrales Material, z.B. ein Polymer oder ein sonstiges schichtbildendes Material. Wichtig ist, das das Hüllmaterial biologisch und physiologisch verträglich ist, das Agens während des Transports schützt und in der Zelle zu physiologisch verträglichen Molekülen abgebaut wird und inert gegenüber dem Agens ist, d.h. mit dem Agens nicht reagiert. Geeignete Hüllmaterialien sind bekannt und werden in vielfältiger Form angeboten. Für die Umhüllung von Nukleinsäuren sind kationische Hüllmaterialien bevorzugt, während für die Umhüllung anderer Agentien, wie Proteine, Wirkstoffe oder Tracer kationische oder neutrale Hüllmaterialien verwendet werden können.

In einer Ausführungsform der vorliegenden Erfindung, insbesondere wenn das Agens eine Nukleinsäure ist, wird als Hüllmaterial ein kationisches Polymer verwendet. Es wurde gefunden, dass kationisch geladene Teilchen von der Zelle leichter aufgenommen werden können als neutrale oder anionisch geladene Teilchen, allerdings können sie auch eher unspezifische Anlagerungen fördern. Zur Umhüllung von Nukleinsäuren als aktiven Bestandteilen sind kationische Hüllmaterialien bevorzugt, da sich Nukleinsäuren sehr einfach mit kationischen Substanzen umhüllen und schützen lassen. Entsprechende Verfahren sind dem Fachmann wohlbekannt.

Das Hüllmaterial kann eine natürlich vorkommende, synthetische oder kationisch derivatisierte natürliche Substanz sein, z.B. ein Lipid oder ein Polymer oder Oligomer. Ein Beispiel für ein natürliches Oligomer ist Spermin. Beispiele für synthetische Polymere sind stickstoffhaltige biologisch abbaubare Polymere, insbesondere solche mit protonierbaren Stickstoffatomen. Besonders geeignet sind Polyethylenimine, insbesondere verzweigte Polyethylenimine, die im Handel erhältlich sind. Geeignet ist beispielsweise ein verzweigtes Polyethylenimin mit einem mittleren Molekulargewicht von 25 kDa, das kommerziell verfügbar ist. Es wurde gefunden, dass dieses Polymer in Kombination mit dem Zielfindungsliganden sehr verträglich ist. Als natürliches ggf. derivatisiertes schichtbildendes Hüllmaterial können auch Lipide, insbesondere kationische oder neutrale Lipide, eingesetzt werden. Lipide sind in vielen Varianten verfügbar und können z.B. zur Bildung von Liposomen eingesetzt werden. Besonders geeignet ist ein kationisch derivatisiertes Lipid, das unter der Bezeichnung Genzyme Lipid 67 erhältlich ist. Weniger geeignet sind auf Zuckermolekülen aufgebaute Polymere wie Stärke oder Stärkederivate, die daher nicht als erfindungsgemäßes Hüllmaterial verwendet werden.

Für andere Agentien, wie Proteine, Wirkstoffe oder Tracer, gibt es eine Anzahl geeigneter Polymere, die dem Fachmann bekannt sind. Geeignet sind solche, die biologisch verträglich sind, und die zumindest in Kombination mit dem erfindungsgemäßen Prostacyclinanalogon nicht entzündlich oder in sonstiger Weise schädlich für die Zelle sind und das Agens, sobald es am Zielort, in der Zelle, angekommen ist, freigeben.

Der aus Hüllmaterial und Agens bestehende Agenskomplex kann z.B. aus an sich bekannten Nanopartikeln oder Nanokapseln, Liposomen etc. bestehen, deren Herstellung wohlbekannt ist. Geeignet ist beispielsweise die Verkapselung in biologisch abbaubare Polymere mit einstellbarer Freisetzung wie Polylactid und/oder Polyglycolid. Das Hüllmaterial kann dabei so ausgewählt werden, dass die Freisetzung des Agens in vorbestimmter Art und Weise erfolgt. Derartige Hüllmaterialien sind in der Literatur vielfach beschrieben und der Fachmann kann aus einer Vielzahl von Materialien das jeweils am besten geeignete auswählen.

Das aktive Mittel wird in an sich bekannter Weise mit dem Hüllmaterial umhüllt oder darin verkapselt. Dieser Komplex aus Agens und umhüllendem Material wird im Folgenden auch als "Agens-Komplex" bezeichnet. Unter "umhüllen" wird im Zusammenhang mit der vorliegenden Erfindung verstanden, dass das Agens soweit durch das Polymer von der physiologischen Umgebung abgeschirmt wird, dass es nicht verändert oder abgebaut wird, solange bis es am Zielort angekommen ist. Die Umhüllung kann nur eine Schicht sein, die das Agens umgibt, es kann auch ein Liposom oder Nano- oder Mikroteilchen sein, in dem das Agens eingebettet oder eingeschlossen ist. Auch kann es durch Komplexierung eingeschlossen sein. Verschiedene Formen der Verkapselung oder Umhüllung von Agentien sind dem Fachmann bekannt und können für das erfindungsgemäße Konjugat eingesetzt werden, solange sie die Bindung des Zielfindungsliganden an den Rezeptor und die Einschleusung des Konjugats in die Zelle nicht stören und in der Zelle das Agens freisetzen. Die Umhüllung des Agens mit dem Hüllmaterial bzw. die Herstellung entsprechender Teilchen kann mit üblichen Verfahren erfolgen. In der einfachsten Ausführungsform wird das aktive Mittel, z.B. eine Nukleinsäure, mit dem Hüllmaterial, z.B. einem kationischen Polymer wie Polyethylenimin, ggf. in gelöster Form vermischt.

An die Hüllsubstanz wird entweder vor der Umhüllung oder danach mindestens ein erfindungsgemäß als Zielführungsstruktur verwendetes Prostacyclinanalogon gebunden. Durch die Bindung des Liganden an das Hüllmaterial wird die Aktivität des Agens nicht beeinträchtigt. Die Bindung oder Immobilisierung des bzw. der Liganden darf die Bindefähigkeit an den Rezeptor nicht stören. Verfahren zur Immobilisierung von Liganden sind dem Fachmann bekannt und die bekannten Verfahren können hier angewendet werden. Der Fachmann kann die Eignung einer Kombination von Hüllsubstanz und Ligand in einfacher Weise feststellen, indem er daraus den gewünschten Agens-Komplex herstellt und die Bindefähigkeit des Komplexes mit der Bindefähigkeit des freien Liganden vergleicht. Weiterhin kann die Eignung einer Hüllsubstanz inbezug auf das Agens festgestellt werden, indem die Aktivität des Agens nach Freisetzung bestimmt und mit der des freien Agens vor Umhüllung verglichen wird.

Die Liganden können direkt an das Hüllmaterial gebunden werden, bevor dieses zur Umhüllung des Agens eingesetzt wird. Diese Ausführungsform ist z.B. bevorzugt, wenn das Hüllmaterial ein kationisches Polymer ist und das Agens eine Nukleinsäure ist. Es ist auch möglich zuerst den Agens-Komplex zu bilden und anschließend die Liganden zu binden. Diese Ausführungsform ist bevorzugt, wenn der Agens-Komplex in Form von Nanoteilchen, Nanokugeln oder Liposomen gebildet wird. Ggf. kann die Bindung des Liganden an das Hüllmaterial auch über einen Spacer so erfolgen, dass die bindungsaktive Stelle des Liganden für die Bindung zugänglich ist. Es wird somit ein Konjugat geschaffen, bei dem das aktive Mittel durch die Bindung nicht beeinflusst wird und das mindestens eine Prostacyclinanalogon auf der Oberfläche zur Bindung an den IP₁-Rezeptor frei zur Verfügung steht. Der Ligand, d.h. das Prostacyclinanalogon, kann durch jede Art von Bindung wie kovalente, ionische oder koordinative Bindung, Wasserstoffbrückenbildung etc., an das Hüllmaterial gebunden werden, solange die Bindung ausreichend ist, um den Liganden zu immobilisieren und seine Rezeptorbindefähigkeit nicht beeinträchtigt wird. So kann die Kupplung des Prostacyclinanalogons an das Hüllmaterial z.B. über kovalente oder ionische Bindung direkt oder über einen Spacer erfolgen. Ein dem Fachmann bekannter Spacer ist z.B. Polyethylenglycol (PEG).

Der Kupplungsgrad, d.h. das Ausmaß der Beladung des Konjugats bzw. der umhüllten Teilchen mit Liganden ausgedrückt als Ligand pro Konjugatteilchen, hat Einfluss auf die Freisetzung des Agens und damit auf die Aktivität des Agens in der Zelle. Der Anteil der an ein umhülltes Teilchen zu bindenden Liganden sollte bevorzugt nicht zu hoch sein, da ansonsten aufgrund sterischer Hinderung die Adressierung des Rezeptors gestört sein könnte. Der Fachmann kann die geeignete Beladung mit Routineversuchen herausfinden. Der Anteil an Liganden hängt ab von der Art des Hüllmaterials und der Größe der Teilchen.

Es wurde auch gefunden, dass ein hoher Kupplungsgrad dazu führen kann, dass das Agens nur noch unvollständig freigesetzt wird. Wird zur Umhüllung ein kationisches Polymer verwendet, sollte daher der Kupplungsgrad 15 Liganden pro Polymer oder weniger sein. Andererseits muss pro umhülltem Teilchen mindestens ein Prostacyclinanalogon gebunden sein, um die Adressierung zu bewirken.

Pro Konjugat bzw. Teilchen kann jeweils eine Art von Prostacyclinanalogon gebunden werden. Es ist auch möglich, eine Mischung von zwei oder mehr Prostacyclinanaloga zu binden, um gegebenenfalls die Bindefähigkeit und/oder Aufnahme in die Zelle zu verstärken.

Es wurde gefunden, dass das Verhältnis von Hüllmaterial zu aktivem Mittel einen Einfluss auf die Wirksamkeit haben kann. Wenn zu wenig Hüllmaterial vorhanden ist, wird das aktive Mittel nicht ausreichend geschützt. Ist der Anteil des Hüllmaterials zu hoch, kann es einerseits Probleme mit der Verträglichkeit geben und andererseits kann ein zu hoher Anteil an Hüllmaterial dazu führen, dass das aktive Mittel nicht mehr freigesetzt werden kann. In beiden Fällen leidet die Effizienz des Transfers. Der Fachmann kann in wenigen Routineversuchen das jeweils am besten geeignete Verhältnis herausfinden. Als besonders geeignet hat sich ein Verhältnis von Hüllmaterial zu aktivem Mittel im Bereich von 10:1 bis 1:4, bezogen auf Gewicht, erwiesen. Besonders bevorzugt ist ein Verhältnis von Hüllmaterial zu Agens von 4:1 bis 1:4. Wenn das Konjugat eine Nukleinsäure als Agens und Polyethylenimin als Polymer enthält, kann der Anteil des Polymers auch über das molare Verhältnis von Polymer-Stickstoffanateil zu DNA-Phosphatanteil angegeben werden; bevorzugt liegt dieses Verhältnis in einem Bereich von 2 bis 10, besonders bevorzugt von 4 bis 8. Es wurde gefunden, dass der hydrodynamische Durchmesser der Konjugatteilchen bei einem molaren Verhältnis von Polymer-Stickstoffanateil zu DNA-Phosphatanteil von 4 bis 8 in einem Bereich von 50 bis 100 nm liegt, was für die Aufnahmeeigenschaften optimal ist.

Weiterhin wurde festgestellt, dass optimale Konjugate dann erhalten werden, wenn die Ligandenbeladungsdichte an den Umhüllungsgrad angepasst wird. Wenn der Anteil an Hüllmaterial relativ hoch ist, sollte die Beladungsdichte nicht zu groß sein, da sonst das Agens zu stark abgeschirmt wird. Wenn der Anteil an Hüllmaterial im unteren Bereich liegt, kann die Beladungsdichte entsprechend im oberen Bereich liegen.

Es wurde gefunden, dass das erfindungsgemäße Konjugat ein ideales Mittel ist, um aktive Wirkstoffe in Bronchial- und/oder Alveolarepithelzellen einzuschleusen. Während mit den im Stand der Technik beschriebenen Teilchen ohne Targetliganden bzw. Zielführungsliganden nur 5% oder weniger ihr Ziel, die Zelle, erreichen und mit den im Stand der Technik beschriebenen ligandenhaltigen Konjugaten auch nur Anteil unter 50 % oder weniger das Ziel erreichen und die Funktion entfalten kann, konnte in Versuchen für die erfindungsgemäßen Konjugate gezeigt werden, dass mehr als 50 %, häufig 60% und mehr und sogar bis zu 80% des erfindungsgemäßen Konjugats von Zellen aufgenommen werden und ihr Agens freisetzen.

Es wird somit ein Mittel zur Verfügung gestellt, mit dem ein aktiver Bestandteil mit hoher Effizienz in Zielzellen eingeschleust wird, wobei einerseits das aktive Mittel während des Transports in die Zelle gut geschützt ist, so dass der Anteil an aktivem Mittel, der in der Zelle ankommt, sehr hoch ist und andererseits die Aufnahmeeffizienz aufgrund der Struktur des erfindungsgemäßen Konjugats sehr hoch ist.

In einer weiteren Ausführungsform kann das erfindungsgemäße Konjugat noch weiter verbessert werden, indem das mit Hüllmaterial, z.B. einem kationischen Polymer umhüllte Teilchen noch zusätzlich mit Polyethylenglycolketten versehen wird, um die Überlebensdauer in der Lunge weiter zu erhöhen. Aktive Moleküle, wie Nukleinsäure durch PEG-ylierung zu schützen, ist an sich bekannt und die üblichen Verfahren können hier verwendet werden.

Das erfindungsgemäße Konjugat kann zur Behandlung von unterschiedlichen Lungenkrankheiten verwendet werden. Insbesondere ist das erfindungsgemäße Konjugat geeignet, um auf Gen- oder Proteindefekten beruhende Lungenkrankheiten zu heilen oder zu lindern. Ein Beispiel hierfür ist zystische Fibrose. Wie oben ausgeführt, ist es sowohl möglich, das mangelnde oder defiziente Gen in die Zelle zu schleusen, als auch ein von dem mangelnden oder defizienten Gen codiertes Protein in die Zelle zu schleusen.

Ein weiteres Anwendungsgebiet für die erfindungsgemäßen Konjugate ist die Verwendung als Impfstoff. In dieser Ausführungsform ist der aktive Bestandteil des Konjugats entweder ein immunmodulatorisch oder immunologisch aktives Peptid oder Protein oder ein Gen, das ein immunmodulatorisch oder immunologisch aktives Protein oder Peptid codiert [1, 2]. Ein Vorteil dieser Ausführungsform der Erfindung ist es, dass über die Lunge nichtinvasiv ein Impfstoff zugeführt werden kann, z.B. mit einem Vernebler oder über ein Aerosol. Diese Technologie ist unkompliziert, ermöglicht den Einsatz auch dort, wo die Verabreichung von Injektionen aufgrund der hygienischen Verhältnisse oder wegen des Fehlens von entsprechend ausgebildetem Personal problematisch ist und ermöglicht eine unkomplizierte mehrfache Dosierung, um die Immunantwort zu stärken. Darüber hinaus ist die Lunge aufgrund ihrer großen Oberfläche und des Vorhandenseins immunologisch aktiver Zellen gut geeignet für eine Impfung.

Das erfindungsgemäße Konjugat wird bereitgestellt zur Verabreichung in die Lunge. Dazu kann es in an sich bekannter Weise formuliert werden als pharmazeutische Zusammensetzung, die durch Inhalation oder über Vernebelung in die Lunge gebracht wird. Entsprechende Formulierungen sind dem Fachmann bekannt. So kann das Konjugat als Suspension oder Emulsion über einen Vernebler oder als Aerosol, mit einem inerten Gas als Trägermittel hergestellt werden. Es kann auch als Pulver eingesetzt werden.

Die vorliegende Erfindung wird durch die folgenden Beispiele weiter erläutert, ohne auf dieses Beispiel beschränkt zu werden.

### Beispiel 1

Es wurden Konjugate von mit einem Hüllmaterial umhüllten Teilchen und Iloprost bzw. Treprostinil als Zielfindungsstruktur hergestellt und untersucht.

### Materialien und Methoden

Die Bezugsquellen für Chemikalien und Plasmide und die verwendeten Konzentrationen sind wie folgt:
Iloprost, Treprostinil und CRY10449: Cayman Chemicals (Michigan, USA) Verzweigtes Polyethylenimin (durchschnittliches Molekulargewicht 25 kDa), N-Hydroxysulfosuccinimid (sulfo-NHS), Rinderserumalbumin (BSA), Natriumphosphat, Picrylsulfonsäurelösung, 4-(2-Hydroxyethyl)piperazin-1-ethansulfonsäure (HEPES) und Heparansulfat: Sigma Aldrich (Schnelldorf, Deutschland)
PEI wurde in doppelt destilliertem Wasser (Wasser zur Injektion, B. Braun Melsungen AG, Melsungen, Deutschland) verdünnt und der pH mit Salzsäure auf 7 eingestellt.

Natriumphosphat wurde in doppelt destilliertem Wasser auf eine Konzentration von 0,5 mM gelöst und der pH mit Natriumhydroxid auf 7,5 eingestellt.

HEPES wurde in destilliertem Wasser auf eine Konzentration von 0,1 M gelöst und der pH mit Natriumhydroxid auf 7,4 eingestellt.

Heparansulfat wurde in doppelt destilliertem Wasser auf eine Konzentration von 5 mg/ml gelöst.

Ethanol p.a. und 3-(N-Morpholino)propansulfonsäure (MOPS): Merck (Darmstadt, Deutschland)
MOPS wurde in doppelt destilliertem Wasser auf eine Konzentration von 0,1 M gelöst und der pH mit Salzsäure auf 6 eingestellt.

1-Ethyl-3-(3-dimethylaminopropyl)carbodiimidhydrochlorid (EDC) und 5-(und 6-) Carboxyfluoresceinsuccinimidylester (Fluorescein-NHS): Pierce (Rockfort, USA) Dithiothreitol (DTT): Amersham Biosciences (South San Francisco, USA) D-Luciferin: Synchem OHG (Flensberg/Altenburg, Deutschland)
Die Plasmide pCMV-luc, das das *Fotinus pyralis* Luciferasegen unter der Kontrolle des frühen Promotors von Cytomegalovirus (CMV) enthielt und pCpG-luc wurden in *E. coli* propagiert und in hoch gereinigter Form (LPS-Gehalt ≤ 0,1 E.U./µg DNA) von Plasmid Factory GmbH (Bielefeld, Deutschland) bereitgestellt. Die Menge an Supercoil pDNA war ≥ 90% ccc (covalently closed circular) für pCMV-luc und ≥ 98% ccc für pCpG-luc.

### Verwendete Zelllinien

A549-Zellen (Humanalveolarepithelzellen): DSMZ (deutsche Sammlung für Mikroorganismen und Zellkulturen, Braunschweig, Deutschland)
BEAS-2B (menschliche Bronchialepithelzellen), H441 (menschliche Broncholarepithelzellen): ATCC (American Type Culture Collection) 16HBE14o-Zellen: Humanbronchialepithelzellen

A549, BEAS-2B und 16HBE14o-Zelllinien wurden in Minimalessenzialmedium (MEM, Gibco-BRL, Karlsruhe, Deutschland), das mit 10% fötalem Kälberserum (FCS, Gibco-BRL, Karlsruhe, Deutschland) ergänzt war, bei 37°C in einer mit 5% CO₂ ergänzten angefeuchteten Luftatmosphäre gezüchtet. Die H441-Zellline wurde in Roswell Park Memorial Institute Medium 1640 (RPMI1640, Gibco-BRL, Karlsruhe, Deutschland), das mit 10% FCS ergänzt war, bei 37°C in einer mit 5% CO₂ angereicherten angefeuchteten Luftatmosphäre gezüchtet.

### Tiere

14 Wochen alte weibliche BALB/c-Mäuse (Charles River Laboratories Sulzfeld, Deutschland) wurden unter spezifischen pathogenfreien Bedingungen gehalten. Die Mäuse wurden mindestens 7 Tage lang vor den Versuchen akklimatisiert. Alle Tierverfahren waren zugelassen und wurden von der lokalen Ethikkommission kontrolliert und nach den Richtlinien des deutschen Rechts zum Schutz von Tierleben durchgeführt.

### Western-Blot-Analyse

A549-, BEAS-2B- und 16HBE14o-Zellen wurden mit PBS gewaschen und auf Eis in Lysepuffer, der 20 mM Tris-HCl (pH 7,5), 100 mM NaCl, 1 mM EDTA, 1% Triton X-100 und 0,05% Natriumdesoxycholat enthielt, lysiert. 1 mM DTT und Proteaseinhibitor-Cocktail (Roche Diagnostics GmbH, Mannheim, Deutschland) wurden direkt vor der Verwendung frisch zugefügt. Die Proteinkonzentrationen wurden unter Verwendung eines Biorad-Proteinassays (Biorad, München, Deutschland) bestimmt. Für jede Zelllinie wurden 50 µg Protein in SDS-Probenbeladungspuffer verdünnt (62,5 mM Tris-HCI (pH 6,8), 2% SDS, 10% Glycerin, 2% DTT, 0,001% Bromphenolblau), der 5 min gesiedet war, auf einem 7,5% Tris-HCl-Gel (Biorad, München, Deutschland) getrennt und auf eine PVDF-Membran (Millipore, Schwalbach, Deutschland) überführt. Die Membranen wurden mit TBS-T (20 mM Tris-HCI (pH 7,6), 137 mM NaCl, 0,1% Tween-20), das 5% Magermilchpulver enthielt (Sigma Aldrich, Deisenhofen, Deutschland) 1 h bei Raumtemperatur blockiert. Der primäre polyklonale Antikörper (1:500 verdünnt) für den IP₁-Rezeptor (Cayman Chemical, Michigan, USA) wurde über Nacht in 0,5% Magermilch inkubiert. Die Membranen wurden mit TBS-T gewaschen und mit einem sekundären HRP-konjugierten Antikaninchen-Antikörper (1:15.000 verdünnt; Biorad, München, Deutschland) 1,5 h bei Raumtemperatur in 0,5% Magermilch inkubiert. Nach mehreren Waschstufen mit TBS-T erfolgte die Chemilumineszenzdetektion unter Verwendung eines ECL Detection Kit (Pierce, Rockfort, USA) nach der Anleitung des Herstellers.

### Synthese von Fluorescein-BSA-Iloprost (FLUO-BSA-ILO) und Fluorescein-BSA-Treprostinil (FLUO-BSA-TRP)

20 mg (0,3 µmol) BSA wurden in 2,5 ml Natriumphosphatpuffer, pH 7,5, verdünnt und mit einem zehnfach molaren Überschuss an Fluorescein-NHS vermischt. Nach einstündigem Rühren bei Raumtemperatur wurde die Mischung auf einer mit PBS equilibrierten Sephadex G25 MPD-10-Säule (GE Health Care, Uppsala, Schweden) gereinigt. Entweder 0,7 mg (1,8 µmol) ILO oder 0,8 mg (1,8 µmol) TRP wurden in 130 µl Ethanol p.a. gelöst und mit 370 µl MOPS-Puffer, 0,1 M, pH 6, vermischt. 0,5 mg (5 mM) sulfo-NHS (in MOPS-Puffer) und 0,2 mg (2 mM) EDC (in MOPS-Puffer) wurden zugegeben und 15 min bei Raumtemperatur gerührt. Danach wurden 5 µl (20 mM) DTT (in destilliertem Wasser) zugefügt und sofort 3 mg (45,2 nmol) FLUO-BSA in 190 µl und 210 µl Phosphatpuffer 0,5 M in die Reaktionsmischung pipettiert. Nach zweistündigem Rühren bei Raumtemperatur wurde die Mischung auf einer mit PBS equilibrierten Sephadex G25 MPD-10-Säule (GE Health Care, Uppsala, Schweden) gereinigt. Die BSA-Mengen wurden quantitativ ausgewertet mit einem Biorad Proteinassay unter Verwendung einer BSA-Standardkurve. Die Kupplungseffizienz der End- und Zwischenprodukte wurde mit TNBS-Assay [21] bestimmt und die Extinktion bei 495 nm gemessen. Der Kupplungsgrad von BSA-ILO und BSA-TRP ergab sich als 10 mol ILO oder TRP pro mol BSA.

### Synthese von mit Iloprost gepfropften PEI-Polymeren (PEI-g-ILO)

Verschiedene Kupplungsgrade von PEI-g-ILO wurden synthetisiert durch Variation der EDC-Mengen, die der Reaktionsmischung zugegeben wurden. 1 mg (2,8 µmol) ILO wurde in 100 µl Ethanol p.a. verdünnt, mit 68 nmol PEI in 900 ml HEPES-Puffer, 0,1 M, pH 7,4, und 1 mg (5 mM) sulfo-NHS gemischt. Verschiedene Mengen an EDC wurden auf eine Endkonzentration von 25 mM, 50 mM, 60 mM bzw. 100 mM zugegeben und 4 h lang unter Rühren bei Raumtemperatur inkubiert. Die Reaktionsmischung wurde an einer mit doppelt destilliertem Wasser equilibrierten Sephadex G25 MPD-10-Säule (GE Health Care, Uppsala, Schweden) gereinigt. Die Konzentration von PEI wurde mit einem CuSO₄-Test gemäß Ungaro et al. [22] bestimmt. ¹H-1D-NMR-Spektren von PEI-g-ILO wurden in D₂O auf einem Brooker AV 250 MHz Spektrometer aufgezeichnet. Die Kupplungsgrade von PEI-g-ILO wurden durch Integration des breiten Multipletts von PEI (CH₂-CH₂-NH-) bei δ (1H) = 2,5 bis 3,1 ppm und des Singuletts der endständigen Methylgruppe von ILO (-C≡-C-CH₃) bei δ (1H) = 1,73 ppm berechnet. Die kovalente Konjugation von ILO an PEI führte zu vier verschiedenen Kupplungsgraden (F_{ILO} (mol ILO/mol PEI) = 2, 5, 8, 16). PEI-g-ILO-Konstrukte wurden in kleine Aliquots aufgeteilt, schockgefroren in flüssigem Stickstoff und bis zur weiteren Verwendung auf -80°C gehalten.

### Inkubationsversuch mit FLUO-BSA-ILO und FLUO-BSA-TRP

Die Rezeptorbindung/Aufnahme von FLUO-BSA-ILO wurde untersucht in A549-, H441-, 16HBE14o- und BEAS-2B-Zellen. Für die FACS-Messversuche wurden 24 Stunden vor Zugabe der Konjugate 100.000 Zellen/Napf in 24-Napfplatten ausgesät (TPP, Trasadingen, Schweiz). FLUO-BSA-ILO, FLUO-BSA-TRP und FLUO-BSA-Konjugate wurden in MEM auf eine Konzentration von 0,5 µM verdünnt und die Zellen 4 h bei 37°C inkubiert. Nach dem Waschen der Zellen mit PBS wurden die Zellen aus den Näpfen mit Trypsinbehandlung entnommen und die FACS-Messungen wurden durchgeführt unter Verwendung eines Beckton-Dickinson-FACS-Scans (San Jose, USA). Für die konfokale Laserrastermikroskopie wurden die Versuche durchgeführt auf Trägern mit 4 Kammern von BD Falcon Culture (BD Biosciences San Jose, USA) mit 25.000 Zellen pro Kammer. Die Inkubation von FLUO-BSA-ILO und FLUO-BSA wurde durchgeführt wie vorher beschrieben. Die Zellen wurden gespült und in 4% Paraformaldehyd fixiert und anschließend wurden die Zellkerne mit 0,33 µM DAPI (4',6-Diamidino-2-phenylindol) und F-Actin mit Alexafluor® 568 Falloidin (Invitrogen GmbH, Karlsruhe, Deutschland) unter Verwendung von Standardprotokollen angefärbt. Die Träger wurden mit Medium (Vectashield, Vector Laboratories Inc., Berlingame, USA) bedeckt und Bilder wurden aufgenommen mit einem konfokalen Laserrastermikroskop (Leica, Solms, Deutschland).

### Versuch zur Hemmung der Bindung von FLUO-BSA-ILO mit CAY10449

Die Hemmung der Rezeptorbindung/Aufnahme von FLUO-BSA-ILO wurde untersucht an 16HBE14o-Zellen. 24-Napfplatten wurden vorbereitet, wie vorher beschrieben. CAY10449 wurde in MEM verdünnt auf Konzentrationen von 15 µM, 30 µM und 150 µM und 15 min bei 37°C inkubiert. Sofort danach wurde FLUO-BSA-ILO und FLUO-BSA auf eine Endkonzentration von 25 nM zugegeben und mit den Zellen 4 h bei 37°C inkubiert. Die Bindung/Aufnahme wurde unter Verwendung von FACS gemessen.

### Herstellung der Genvektorteilchen

Luciferasereportergen enthaltendes Plasmid (pCMV-luc), PEI bzw. PEI-g-ILO wurden getrennt in 25 µl doppelt destilliertem Wasser verdünnt. Verschiedene N/P-Verhältnisse (molares Verhältnis von PEI-Stickstoff zu DNA-Phosphat) wurden getestet. Die pCMV-luc-Lösung wurde einem gleichen Volumen der Polymerlösung zugefügt und vorsichtig vermischt, indem achtmal auf- und abpipettiert wurde, was zu Teilchen mit einer Konzentration von 20 µg pCMV-luc/ml führte. Gentransferteilchen wurden 20 min bei Raumtemperatur inkubiert.

### Teilchengrößenmessung

Die Teilchengröße (bestimmt mit dynamischer Lichtstreuung) wurde gemessen unter Verwendung eines Zeta PALS/Zeta Potential Analyzers (Brookhaven Instruments Corporation, Wien, Österreich). Genvektorteilchen wurden wie oben beschrieben erzeugt. Die folgenden Einstellungen wurden verwendet: 5 Durchläufe mit 1 min Messung pro Probe; Viskosität für Wasser 0,89 cP; Ref. Index 1.330; Temperatur 25°C.

### DNA-Retardationstest

PEI/pCMV-luc und PEI/g-ILO/pCMV-luc-Genvektorteilchen mit verschiedenen Kupplungsgraden mit N/P = 4 wurden in doppelt destilliertem Wasser wie oben beschrieben hergestellt. 5 µl jeder Teilchenlösung wurden entweder mit 2 µl doppelt destilliertem Wasser oder 2 µl Heparansulfatlösung (5 mg/ml) vermischt. Nach 45-minütiger Inkubation wurden Proben mit 1 µl Beladungspuffer (0,25% Bromphenolblau, 0,25% Xylolcyanol FF, 30% Glycerin in Wasser) vermischt, in einzelne Näpfe eines 0,8% Agarosegels geladen und mit Agarosegelelektrophorese mit 125 V 1 h lang getrennt. Das Gel wurde mit Ethidiumbromid angefärbt und die DNA-Banden wurden unter UV-Licht sichtbar gemacht.

### In-vitro-Transfektionsstudien

A549-, 16HBE14o- und BEAS-2B-Zellen wurden 24 h vor der Transfektion mit einer Dichte von 100.000 Zellen/Napf in 24-Napfplatten (TPP, Trasadingen, Schweiz) gesät und in MEM, das 10% FCS enthielt und mit 0,1% (V/V) Penicillin/Streptomycin ergänzt, gezüchtet. Vor der Transfektion wurden die Zellen mit PBS gewaschen und 450 µl frisches serumfreies Medium wurde pro Napf zugefügt. Anschließend wurden 50 µl der Genvektorteilchen, entsprechend 1 µg pCMV-luc, auf die Zellen pipettiert. Für die Hemmungsversuche wurde CAY10449 zu frischem Medium mit einer Konzentration von 150 µM 15 min vor Zugabe der Genvektorteilchen zugefügt. Nach 4-stündiger Inkubation wurde das Transfektionsmedium durch MEM ersetzt, das 10% FCS enthielt und mit 0,1% (VlV) Penicillin/Streptomycin ergänzt war. 24 h nach der Transfektion wurde die Luciferaseaktivität gemessen unter Verwendung eines Wallac Victor² 1420 Multilabel Counters (Perkin Elmer, Boston, USA) gemäß Huth et al. [23]. Die Ergebnisse wurden auf einen Gesamtzellproteingehalt normalisiert unter Verwendung eines Biorad-Proteinassays und von BSA als Proteinstandard.

### In-vivo-Gentransferstudien

Um Genvektorteilchen für die Aerosolabgabe an Mäuse herzustellen, wurden pCpG-luc und PEI oder PEI-g-ILO F_{ILO} = 5 jeweils mit 4,0 ml Wasser zur Injektion (B. Braun Melsungen AG, Melsungen, Deutschland) verdünnt, was zu Konzentrationen von 250 µg/ml pCpG-luc bzw. 130,4 µg/ml PEI führte (entsprechend einem N/P-Verhältnis von 4). Die pCpG-luc-Lösung wurde zu der PEI-Lösung pipettiert, durch 8-maliges Auf- und Abpipettieren vermischt, was eine endgültige DNA-Konzentration von 125 µg/ml lieferte. Die Teilchen wurden 20 min bei Raumtemperatur vor der Verwendung inkubiert. Die Teilchen wurden unter Verwendung einer PARI Turboboy®N Inhalationsvorrichtung mit einem PARI LC+ Vernebler (PARI GmbH, Starnberg, Deutschland), der mit einer vertikalen Gesamtkörperaerosolvorrichtung gemäß Rudolph et al. [24] verbunden war, vernebelt. Nach 24 h wurden Mäuse anästhesiert und eine pulmonale Verabreichung von D-Luciferinsubstrat (1,5 mg/50 µl PBS pro Maus) erfolgte durch Schnüffeln [25]. Nach 10 min wurde die Biolumineszenz gemessen (IVIS 100 Imaging System; Xenogen, Alameda, USA) unter Verwendung der folgenden Kameraeinstellungen: Gesichtsfeld 10, F1 f-stop, High Resolution Binning und Belichtungszeit 10 min. Um die Reportergenexpressionsgrade in den Lungen zu bestätigen, wurden die Mäuse nach der in-vivo-Biolumineszenzaufnahme durch zervikale Dislokation getötet. Nach Öffnen des Bauchfells durch Einschnitt entlang der Mittellinie wurden die Lungen der Tiere seziert und mit PBS perfundiert. Die Lungen wurden in flüssigem Stickstoff schockgefroren und in gefrorenem Zustand homogenisiert. Nach Zugabe von 400 µl Lysepuffer (250 mM Tris, pH 7,8, 0,1% Triton X-100, Roche Complete Proteaseinhibitorcocktail-Tabletten) und 20-minütiger Inkubation auf Eis wurde die Luciferaseaktivität im Überstand gemessen unter Verwendung eines Lumat LB9507 Röhrenluminometers (EG & G Berthold, München, Deutschland). Rekombinante Luciferase (Roche Diagnostics GmbH, Mannheim, Deutschland) wurde als Standard verwendet, um die Menge an Luciferase, die im Lungengewebe exprimiert wurde, zu berechnen.

### Auf MTT basierender Test

Die Toxizität von PEI/pCMV-luc oder PEI-g-ILO F_{ILO} = 5/pCMV-luc-Teilchen wurde ausgewertet an 16 HBE14o-Zellen mit einem N/P-Verhältnis von 4. Die Zellen wurden 24 h vor dem Versuch mit einer Dichte von 80.000 Zellen/Napf in eine 24-Napfplatte gesät. Die Transfektion erfolgte wie vorher beschrieben. Nach 4 h wurde die Transfektionsmischung durch 400 µl Medium ersetzt und ein auf MTT basierender Test wurde durchgeführt unter Verwendung des Cell Proliferation Kit 1 (Roche Diagnostics GmbH, Mannheim, Deutschland) nach der Anleitung des Herstellers. Unbehandelte Zellen wurden als Referenz verwendet, indem die entsprechende Absorption auf 100% lebensfähige Zellen eingestellt wurde.

### Sammeln von Serum und Analyse der Cytokinkonzentration

24 h nach der Abgabe des Aerosols wurden Blutproben von den Mäusen entnommen und bei 4°C über Nacht aufbewahrt. Das Blut wurde zentrifugiert und das Serum gesammelt. Die quantitative Bestimmung von Interleukin-12 (IL-12) und interferon-γ (IFN-γ) erfolgte unter Verwendung des Maus-IL-12 (P40/P70) und Maus-INF-γ-ELISA Kits (Ray Biotech, Norcross, USA) nach der Anleitung des Herstellers. Unbehandelte Mäuse wurden als Referenz verwendet, indem die entsprechende Konzentration auf 1 gesetzt wurde.

### Statistische Analyse

Die Ergebnisse sind angegeben als Mittelwert ± Standardabweichung. Statistisch signifikante Unterschiede wurden ausgewertet mit dem nicht gepaarten Student-T-Test. p < 0,01 wurde als signifikant angesehen.

### Ergebnisse

### Bestätigung der IP₁-Rezeptorexpression in Lungenzellen mit Western Blot

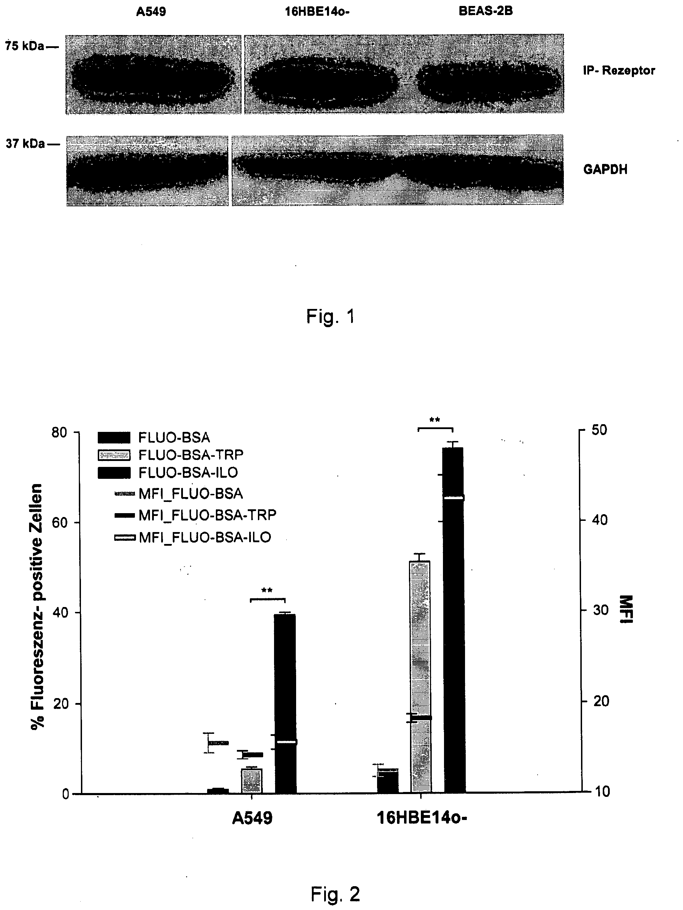

Die Expression von IP₁-Rezeptor in menschlichen Alveolar-(A549)- und Bronchial-(BEAS-2B, 16HBE14o-)epithelzellen wurde mit Western-Blot-Analyse bestätigt. Eine Proteinbande bei 47 kDa konnte nachgewiesen werden (Fig. 1), was der glycosilierten Form des IP₁-Rezeptorproteins, exprimiert auf der Zellmembran entspricht [26]. Es wurde daher untersucht, ob die gezielte Ansteuerung des IP₁-Rezeptors für die Zuführung von Proteinen oder Genen möglich ist.

### Ansteuerung von Lungenzellen mit unterschiedlichen IP₁-Rezeptorliganden

Um die Zielführung zu dem IP₁-Rezeptor für rezeptorvermittelten Gentransfer zu untersuchen, wurden TRP und ILO chemisch an Fluorescein-markiertes Rinderserumalbumin (FLUO-BSA) gekuppelt, das als Modellsubstanz diente. Während die Inkubation von A549- und 16HBE14o-Zellen mit FLUO-BSA zu unspezifischer Hintergrundbindung führte, führte die Inkubation mit FLUO-BSA-TRP und FLUO-BSA-ILO zu 5,5 ± 0,5% bzw. 39,3 ± 0,6% positiven A549- und 51 ± 1,8% bzw. 76,1 ± 1,4% positiven 16HBE14o-Zellen (Fig. 2). Die mittlere Fluoreszenzintensität (MFI) von A549- und 16HBE14o-Zellen war signifikant höher nach Inkubation mit FLUO-BSA-ILO als nach Inkubation mit FLUO-BSA-TRP. Diese Ergebnisse zeigen, dass TRP und ILO eine erfolgreiche Bindung der Modellsubstanz FLUO-BSA an Lungenzellen vermitteln können, dass aber ILO der wirksamere Zielführungsligand ist.

### Spezifität der FLUO-BSA-ILO-Bindung an verschiedene Lungenzelllinien

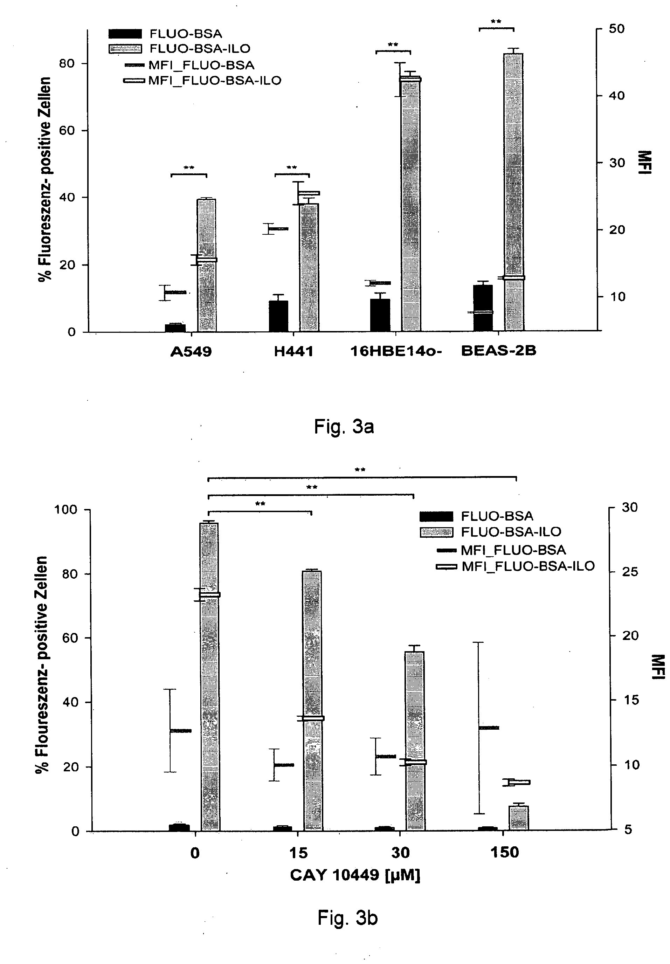

ILO wurde weiter untersucht als Zielführungsligand an zusätzlichen Lungenzelllinien aufgrund der besseren Zellbindung/Aufnahme im Vergleich zu TRP. Zusätzlich zu A549- und 16HBE14o-Zellen erzeugte die Inkubation von H441- und BEAS-2B-Zellen mit FLUO-BSA-ILO eine signifikant höhere Anzahl (p < 0,01) von positiven Zellen und MFI als die Kontrolle FLUO-BSA (38,0 ± 1,8%) bzw. 82,7 ± 1,6% gegenüber 9,1 ± 1,9% bzw. 13,7 ± 1,2% (Fig. 3A). Diese Wirkung war deutlicher an menschlichen Bronchialepithelzellen (16HBE14o-, BEAS-2B) als bei Clara-(H441)- oder Alveolar-(A549)-Epithelzellen. Diese Ergebnisse zeigen die unterschiedliche Zelloberflächenexpression von IP₁-Rezeptor in menschlichen Lungenzelltypen.

Um die Rezeptorspezifität der beobachteten Bindung von FLUO-BSA-ILO in Lungenzellen zu bestätigen, wurden 16HBE14o-Zellen mit FLUO-BSA-ILO in Gegenwart von steigenden Mengen CAY10449 inkubiert. Von dieser Verbindung wurde bereits früher berichtet, dass sie ein hochspezifischer potenter Antagonist von Human-IP₁-Rezeptor ist [27, 28]. 16HBE14o-Zellen wurden mit 25 nM FLUO-BSA-ILO zusammen mit steigenden Konzentrationen von CAY10449 inkubiert. Die Zugabe von CAY10449 führte zu einer signifikanten dosisabhängigen Verminderung (p < 0,01) sowohl der Anzahl von Fluoreszenz-positiven Zellen als auch des MFI (Fig. 3B). Bei der höchsten verwendeten Konzentration von CAY10449 nahm die Anzahl der Fluoreszenz-positiven Zellen von 95,7 ± 0,7% auf 7,4 ± 0,9% ab. Die Zellen, die mit FLUO-BSA-Konjugaten inkubiert worden waren, wurden als Kontrollen verwendet und zeigten keine Wirkung bei Zugabe von CAY10449. Ähnliche Ergebnisse wurden erhalten bei kompetitiven Versuchen mit einem Überschuss von unkonjugiertem ILO.

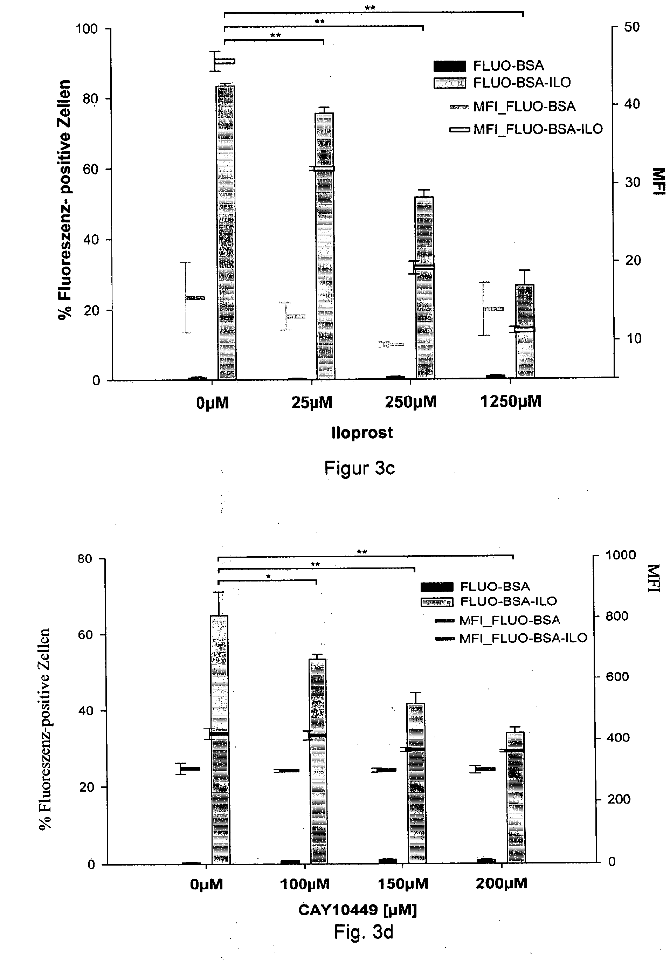

FACS-Messungen zusammen mit Inhibitionsversuchen deuten auf eine Zelltypabhängige Zelloberflächenexpression von IP₁-Rezeptor auf Lungenepithelzellen. Um weiter zu testen, ob ILO eine intrazelluläre Aufnahme von FLUO-BSA-ILO vermittelt, wurden zusätzliche Versuche durchgeführt unter Verwendung von konfokaler Laserrastermikroskopie. 16HBE14o-Zellen wurden entweder mit 0,5 µM FLUO-BSA oder FLUO-BSA-ILO inkubiert. Die Sichtbarmachung der Zellen durch konfokale Mikroskopie zeigte eine klare Zelloberflächenbindung und anschließende intrazelluläre Aufnahme von FLUO-BSA-ILO-Konjugaten (Fig. 3C), wohingegen keine Aufnahme von FLUO-BSA beobachtet werden konnte.

### Charakterisierung von PEI und PEI-g-ILO-Genvektorteilchen

ILO wurde an PEI über Carbodiimidchemie mit steigendem Kupplungsgrad gekuppelt F_{ILO} = 2, 5, 8 bzw. 16 und die Größe der entstehenden Genvektorteilchen wurde mit dynamischer Lichtstreuung gemessen (Tabelle 1). Teilchen mit einem Kupplungsgrad F_{ILO} = 2 und 5 mit einem N/P-Verhältnis von 4 bis 8 zeigten hydrodynamische Durchmesser von 50 bis 100 nm, die mit PEI-Genvektoren vergleichbar waren. Teilchen, die mit PEI N/P 2, PEI-g-ILO F_{ILO} = 2 N/P 2 bis 3 und PEI-g-ILO F_{ILO} = 16 N/P 4 hergestellt wurden, waren nicht stabil und fielen aus. Teilchen, die kleiner als 150 nm waren, zeigten eine Polydispersität von < 0,2.

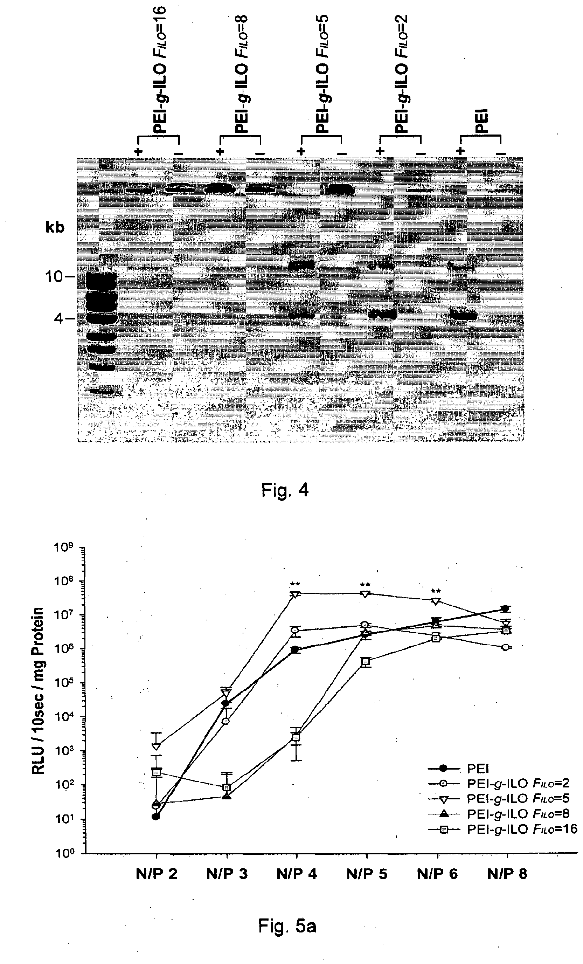

Als Nächstes wurde die DNA-Bindungsaffinität der PEI-g-ILO-Konstrukte bestimmt. Teilchen mit N/P 4 wurden hergestellt und ein DNA-Freisetzungstest wurde durchgeführt (Fig. 4). Für PEI, PEI-g-ILO F_{ILO} = 2 und PEI-g-ILO F_{ILO} = 5 gab es eine vollständige Freisetzung der DNA nach Zugabe von Heparansulfat. Bei einem hohen Kupplungsgrad von 16 konnte nur eine teilweise Freisetzung der DNA beobachtet werden, was auf eine stärkere Bindung der Polymere an das Plasmid hindeutet. Ein Kupplungsgrad von 16 oder mehr ist daher weniger bevorzugt.

### In-vitro-Transfektionseffizienz

Eine erhöhte Bindung und Aufnahme von FLUO-BSA-ILO in verschiedenen Lungenzellen und die Möglichkeit, PEI-g-ILO/pCMV-luc-Teilchen zu bilden, war Anlass, ILO als Liganden weiter zu untersuchen, um den Gentransfer in vitro zu verbessern. 16HBE14o-Zellen wurden mit PEI-g-ILO-Genvektoren transfiziert und mit unmodifiziertem PEI als Kontrolle verglichen. Die Gentransfereffizienz nahm mit dem N/P-Verhältnis zu. Der höchste Grad an Genexpression fand sich bei N/P 4 und F_{ILO} = 5. Bei diesen optimierten Bedingungen war die Genexpression signifikant 46-fach höher als für PEI (Fig. 5A). Die Teilchenbildung bei höheren N/P-Verhältnissen (> 4) führte zu keinem weiteren Anstieg der Genexpression. PEI-g-ILO-Konjugate mit anderen Kupplungsgraden führten entweder zu geringeren oder gleichen Transfektionsgraden im Vergleich zu PEI.

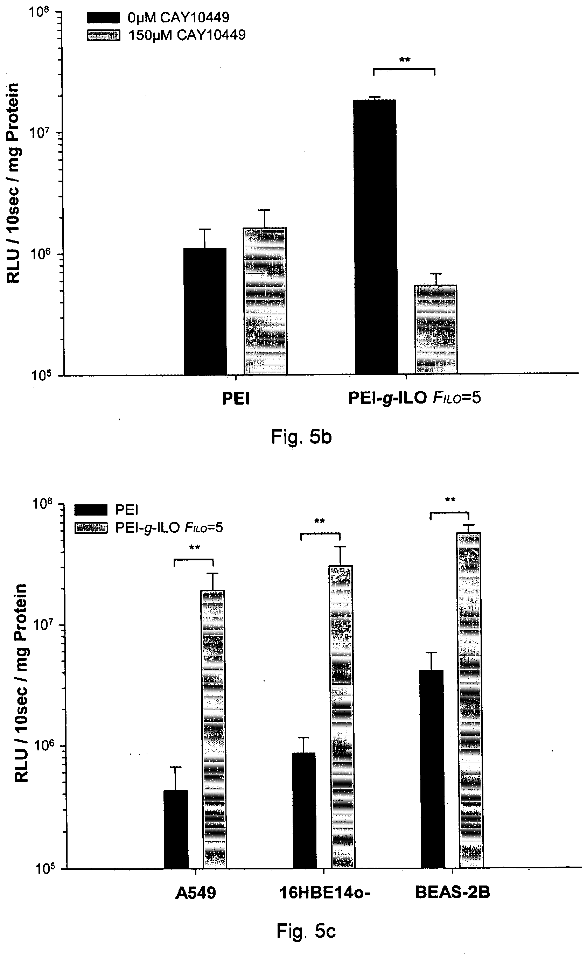

Die Versuche zur kompetitiven Hemmung mit CAY10449 wurden durchgeführt, um den Rezeptor-vermittelten Gentransfer von PEI-g-ILO-Genvektoren zu bestätigen. 16HBE14o-Zellen wurden entweder mit PEI oder PEI-g-ILO F_{ILO} = 5 Genvektoren mit N/P 4 in Gegenwart oder Abwesenheit von 150 µM CAY10449 transfiziert. Die mit PEI-g-ILO F_{ILO} = 5 beobachtete Genexpression war signifikant (p < 0,01) 33-fach reduziert gegenüber PEI (Fig. 5B). Bei Zellen, die mit PEI transfiziert waren, wurde keine Wirkung durch CAY10449 beobachtet.

Außerdem wurde PEI-g-ILO F_{ILO} = 5 auch an A549- und BEAS-2B-Zellen getestet. Bei optimierten Bedingungen war die durch PEI-g-ILO F_{ILO} = 5 vermittelte Expression gegenüber PEI bei A549- bzw. BEAS-2B-Zellen 45-fach bzw. 14-fach höher (Fig. 5C).

### Untersuchungen zur Genfreisetzung in-vivo

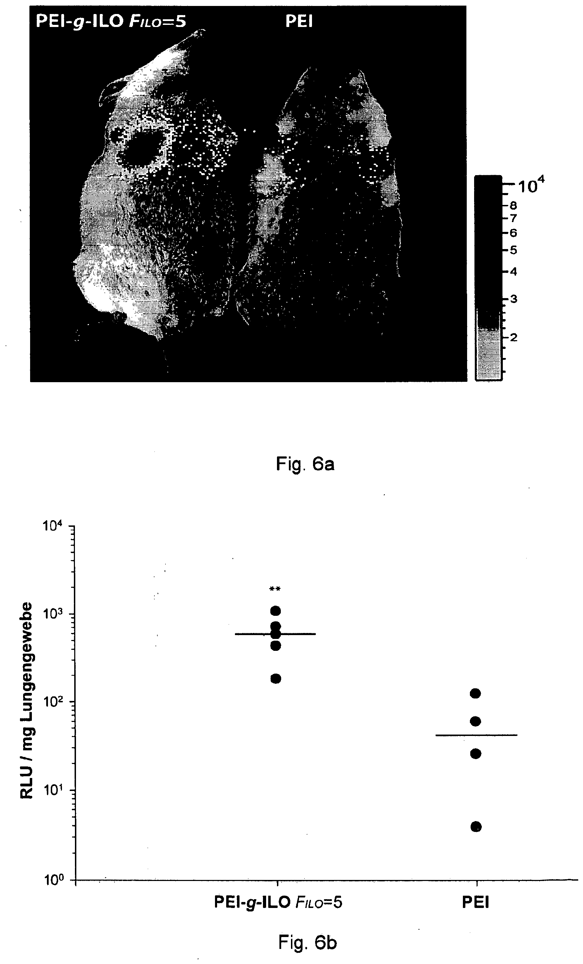

PEI-g-ILO F_{ILO} = 5 und PEI-Genvektorteilchen wurden der Lunge von BALB/C-Mäusen über Aerosol zugeführt und die Genexpression wurde 24 h nach der Genabgabe analysiert. Die Messung der Luciferasegenexpression gegenüber der in-vivo-Biolumineszenzabbildung zeigt ein starkes Signal in den Lungen von Mäusen, die mit PEI-g-ILO F_{ILO} = 5 Genvektoren behandelt worden waren, war aber an der Nachweisgrenze für PEI-Genvektoren (Fig. 6A). Um die Luciferase pro mg Lungengewebe quantitativ auszuwerten, wurden die Mäuse getötet und die Lungen isoliert. Die Luciferaseexpression, die im homogenisierten Lungengewebe gemessen wurde, war signifikant 14-fach höher für PEI-g-ILO F_{ILO} = 5 Genvektoren als für PEI-Genvektoren (Fig. 6B).

### Toxizität in vitro und in vivo

Die in-vitro-Lebensfähigkeit nach Anwendung der Genvektorteilchen (PEI-g-ILO F_{ILO} = 5/pCMV-luc oder PEI/pCMV-luc) wurde gemessen unter Verwendung eines MTT-Assays. Im Vergleich zu PEI konnte kein Anstieg an Cytotoxizität beobachtet werden (86,0 ± 10,1% Zelllebensfähigkeit für PEI-g-ILO F_{ILO} = 5 gegenüber 89,2 ± 3,2% für PEI). Für die Bestimmung der in-vivo-Toxizität und Entzündung wurde Serum von behandelten Mäusen erhalten und es wurden die Entzündungscytokine, einschließlich Interleukin-12 (IL-12) und Interferon-γ (INF-γ) gemessen. Ähnlich wie bei den in-vitro-MTT-Ergebnissen konnte kein signifikanter Anstieg bei den Cytokinen 24 h nach Genabgabe mit ELISA nachgewiesen werden.

Mit den obigen Versuchen konnte gezeigt werden, dass das Prostaglandin-I₂-Analog ILO, ein IP₁-Rezeptoragonist als Zielführungsligand verwendet werden kann, um den Gentransfer kationischer Polymere, wie PEI in Lungenzellen in vitro und in vivo zu verbessern. Es wurde gefunden, dass mit den erfindungsgemäßen Konjugaten, die ein Prostaglandin-I₂-Analogon als Zielführungsligand und ein kationisches Polymer als Umhüllung für eine aktive Substanz enthalten, die Genexpression signifikant verbessert werden kann. So wurde in der Studie gezeigt, dass die Reportergenexpression signifikant erhöht war in menschlichen Alveolar-(A549)- und Bronchialepithelzellen (16HBE14o-, BEAS-2B) und zwar um das bis zu 46-Fache. Außerdem war die Luciferaseaktivität in der Lunge von Mäusen nach Aerosolbehandlung signifikant, nämlich 14-fach höher als bei PEI.

ILO und TRP sind Agonisten des menschlichen IP₁-Rezeptors [29]. Beide sind zugelassen zur Behandlung von pulmonalem arteriellem Hochdruck über Aerosolinhalation bzw. i.v.-Applikation [20, 30, 31]. IP₁-Rezeptoren werden in der Lunge von Menschen und Mäusen exprimiert [15, 32-34] und IP₁-Rezeptor/Ligandkomplexe werden in die Zelle internalisiert [35, 36]. Erfindungsgemäß werden diese Eigenschaften ausgenutzt, um ein verbessertes System zur Einschleusung von Agentien in Lungenzellen zur Verfügung zu stellen.

Mit Western Blot konnte die IP₁-Rezeptorexpression in verschiedenen Lungenzellarten bestätigt werden. Um die IP₁-Rezeptorexpression auf der Zelloberfläche von Lungenzellen detaillierter zu charakterisieren, wurden Fluorescein-markierte BSA-Konjugate, die entweder an ILO oder TRP gekuppelt waren, synthetisiert. Beide Konstrukte wurden dann mit Alveolar-(A549)- und Bronchial-(16HBE14o)-Epithellzelllinien inkubiert und die Zellbindung mit Durchflusscytometrie analysiert. Die Ergebnisse zeigen, dass IP₁-Rezeptoren auf jeder der getesteten Zelllinien vorhanden sind. ILO zeigt jedoch eine höhere Zelloberflächenbindung als TRP und ILO wurde daher als Zielführungsligand in allen nachfolgenden Versuchen verwendet. Die Spezifität der Bindung des IP₁-Rezeptors wurde gezeigt durch Inhibitionsversuche mit dem spezifischen IP₁-Rezeptorantagonisten CAY10449 [27, 28, 32] und durch Überschuss von freiem ILO. Um diese Beobachtungen zu bestätigen, wurde eine konfokale Laserrastermikroskopie durchgeführt, die die Bindung von FLUO-BSA-ILO an die Zelloberfläche und die intrazelluläre Aufnahme in 16HBE14o-Zellen zeigte. Diese Ergebnisse belegen daher, dass ILO als Zielführungsligand erfindungsgemäß verwendet werden kann, der die Bindung und intrazelluläre Aufnahme von konjugierten Substanzen, wie FLUO-BSA vermittelt, was eine Vorbedingung für eine rezeptorvermittelte Aufnahme von Genvektornanoteilchen ist.

Für Transfektionsstudien wurde ILO an verzweigtes PEI 25 kDa über eine Amidbindung konjugiert. Die Synthese führte zu Konjugaten mit einem Kupplungsgrad von F_{ILO} = 2, 5, 8 und 16. PEI-g-ILO/pCMV-luc-Teilchen wurden an 16HBE14o-Zellen gescreent und die höchste Transfektionseffizienz wurde bei N/P 4 F_{ILO} = 5 beobachtet, wohingegen ein höherer Kupplungsgrad von 8 bis 16 zu einer geringeren Transfektionsrate führte. Dies könnte auf einer unvollständigen Freisetzung von pCMV-luc bei höheren Kupplungsgraden beruhen, was mit einem DNA-Freisetzungstest beobachtet wurde. Die Freisetzung von pDNA aus PEI/p-DNA-Teilchen wurde bereits als kritischer Parameter für einen erfolgreichen Gentransfer festgestellt [37]. Es kann spekuliert werden, dass eine zusätzliche hydrophobe Wechselwirkung von ILO mit pDNA die pDNA-Bindung erhöhen könnte. Die Messung der Größe verschiedener Teilchen zeigte, dass eine steigende Menge an Ligand zu größeren hydrodynamischen Durchmessern für PEI-g-ILO/pCMV-luc-Teilchen führt bis zu 1 µm. Ähnliche Ergebnisse wurden bei Elfinger et al. erhalten, wenn Clenbuterol an PEI gekuppelt wurde [13]. Teilchen mit PEI-g-ILO F_{ILO} = 5 zeigten hydrodynamische Durchmesser von weniger als 100 nm. Teilchen ähnlicher Größe konnten effizienter internalisiert werden als größere Teilchen, was bereits gezeigt wurde [38]. Die Transfektion von Alveolar-(A549)- und Bronchial-(16HBE14o-, BEAS-2B)-Epithelzellen mit PEI-g-ILO F_{ILO} = 5/pCMV-luc-Teilchen mit N/P 4 führte zu einer 46-fachen Zunahme der Reportergenexpression im Vergleich zu PEI/pCMV-luc-Teilchen mit einem gleichen N/P-Verhältnis in allen getesteten Zelllinien. Die beobachtete verbesserte Genexpression führte nicht zu einem erhöhten Anstieg der metabolischen Toxizität, was mit einem MTT-Test gemessen wurde. Weiterhin wurde die Hypothese des rezeptorvermittelten Gentransfers weiter gestützt durch die Versuche mit einer durch spezifischen Antagonisten vermittelten Hemmung in 16HBE14o-Zellen. Die Zugabe von CAY10449 verringerte die Genexpression auf ein Ausmaß, das mit PEI vergleichbar ist.

Für Tierversuche wurde ein CpG-freies Luciferaseexpressionsplasmid (pCpG-luc) verwendet. Es hat sich erwiesen, dass CpG-freie Plasmide weniger entzündlich wirken als CpG-haltige. Es wurde auch gezeigt, dass sie zu einer höheren und längeren Genexpression in der Lunge führen [39]. Vor den Tierversuchen wurden PEI-g-ILO F_{ILO} = 5/pCpG-luc und PEI/pCpG-luc-Genvektoren vernebelt und verschiedene Fraktionen gesammelt (vernebelt, nicht vernebelt), um die Stabilität der Teilchen zu testen. Sowohl der Gelretardationstest als auch die Teilchengrößenmessungen zeigten keine Veränderung der Teilchen nach Vernebelung im Vergleich zu nicht vernebelten Teilchen. Diese Beobachtungen bestätigten, dass Teilchen nicht negativ beeinflusst wurden durch die Aerosolbildung. Gleiche Ergebnisse wurden bereits berichtet [40]. Nach der Aerosolgenabgabe an die Lungen von Mäusen war die Genexpression signifikant, 14-fach, höher für PEI-g-ILO F_{ILO} = 5/pCpG-luc als für PEI/pCpG-luc-Genvektoren. Die Messung von Interleukin-12 (IL-12) und Interferon-γ (INF-γ) im Mausserum zeigte keinen signifikanten Anstieg dieser Cytokine. Diese Beobachtung stimmt überein mit Gautham et al. [41], wo gezeigt wurde, dass die Aerosolabgabe von PEI-DNA-Teilchen keine höhere Cytokinantwort induziert.

Zusammenfassend ist festzustellen, dass erfindungsgemäß eine neue Zielfindungsstruktur für die Zuführung von Substanzen in die Lunge bereitgestellt wird. Das Potenzial von Prostacyclinanaloga und insbesondere von ILO als Ligand für die Zielführung wurde von den Erfindern erkannt und als "Fähre" für die Verabreichung von Substanzen in Lungenzellen genutzt. Insbesondere sind Prostacyclinanaloga ILO nützlich als Zielführungsliganden für nicht virale Vektoren in Aerosolform. Unter Verwendung von auf Fluorescein basierenden molekularen Konjugaten konnte gezeigt werden, dass der IP₁-Rezeptor ein geeigneter Kandidat für einen rezeptorvermittelten Gentransfer in Lungenzellen ist. Die rezeptorspezifische Bindung und Aufnahme von Molekülkonjugaten konnte sowohl für Alveolar- als auch Bronchialepithelzellen und Clarazellen gezeigt werden. Die erfindungsgemäßen Konjugate führen zu einem spezifischen signifikanten Anstieg der Genexpression in vitro und in vivo. Durch die mehr als 10-fache Erhöhung der Genexpression ist es möglich, die Menge an pDNA und an Genträger zu reduzieren, was die durch DNA oder Träger vermittelte Toxizität und Entzündung senkt.

Die Ergebnisse dieses Beispiels sind in den Figuren 1 bis 7 und Tabelle 1 gezeigt:
Tabelle 1: Physikalische Charakterisierung von PEI/pCMV-luc und PEI-g-ILO/pCMV-luc-Genvektoren unter Verwendung von PEI-g-ILO mit verschiedenen Kupplungsgraden (F_{ILO} = 2, 5, 8, 16) bei verschiedenen N/P-Verhältnissen:
Messungen der Teilchengröße und der Polydispersität (in Klammern). Die Ergebnisse sind angegeben als Mittelwert ± Standardabweichung (n = 3).
Fig. 1 Der Western-Blot zeigt die Expression von IP₁-Rezeptorprotein mit 67 kDa in menschlichen Alveolar-(A549)- und Bronchial-(BEAS-2B, 16HBE14o)-Epithelzellen. Jede Bahn wurde mit 40 µg Proteinextrakt beladen.
Fig. 2 Zielfindung des IP₁-Rezeptors mit TRP und ILO an Alveolar-(A549)- und Bronchial-(16HBE14o)-Epithelzelllinien. Die Inkubation von FLUO-BSA, FLUO-BSA-TRP und FLUO-BSA-ILO wurde mit einer Konzentration von 0,5 µM durchgeführt (n = 4): FACS-Messungen. Die Ergebnisse sind angegeben als Mittelwert ± Standardabweichung. **bedeutet statische Signifikanz mit p < 0,01.

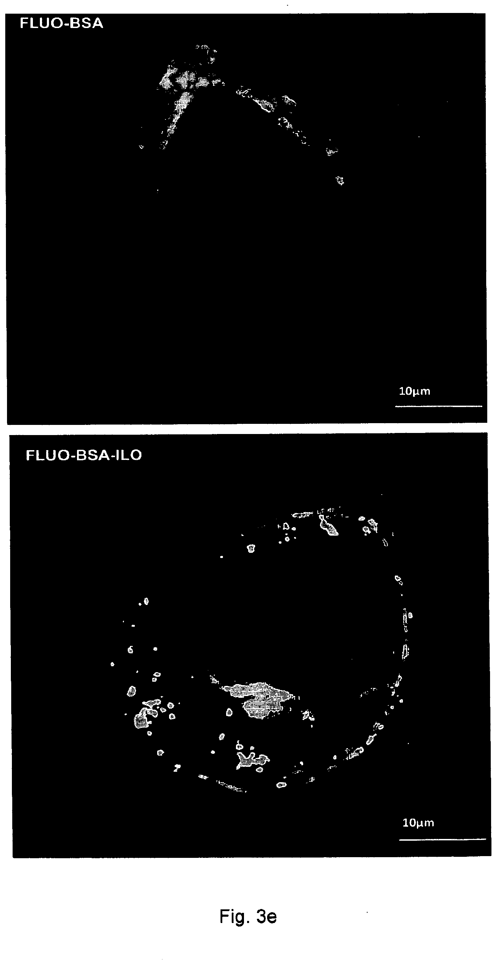

Fig. 3 Verteilung von IP₁-Rezeptor und die Rezeptorbindung in Alveolar-(A549)-, Bronchoalveolar-(H441)- und Bronchial-(16HBE14o-, BEAS-2B)-Epithelzellen. Die Inkubation mit FLUO-BSA-ILO und FLUO-BSA wurde durchgeführt mit einer Konzentration von 0,5 µM (n = 4): FACS-Messungen (a). 16HBE14o- (b,c) und A549-(d)-Zellen wurden mit 25 nM FLUO-BSA-ILO und FLUO-BSA zusammen mit steigenden Konzentrationen von CAY10449 (n = 4) inkubiert; FACS-Messungen (b,c,d). Für die konfokale Laserrastermikroskopie wurden 16HBE14o-Zellen mit 0,5 µM FLUO-BSA-ILO und FLUO-BSA inkubiert (e). Die Ergebnisse sind angegeben als Mittelwert ± Standardabweichung. **bedeutet statistische Signifikanz mit p < 0,01.
Fig. 4 DNA-Verzögerungstest für PEI und verschiedene PEI-g-ILO-Konstrukte mit N/P = 4. Polymere, die mit pCMV-luc komplexiert waren, wurden mit (+) und ohne (-) Heparansulfat inkubiert, auf Agarosegel aufgetrennt und unter UV-Licht nach Ethidiumbromidanfärbung sichtbar gemacht.
Fig. 5 Transfektionseffizienz in vitro. Die Transfektion von 16HBE14o-Zellen mit pCMV-luc komplexiert mit verschiedenen PEI-g-ILO-Konstrukten bei verschiedenen N/P-Verhältnissen (n = 4): Messung der Luciferasegenexpression (a), Hemmexperiment von PEI/pCMV-lüc- und PEI-g-ILO F_{ILO} = 5/pCMV-luc-Teilchen mit einem N/P-Verhältnis von 4 mit CAY10449 (n = 3): Messung der Luciferasegenexpression (b). Transfektion von A549, 16HBE14o- und BEAS-2B mit PEI/pCMV-luc und PEI-g-ILO F_{ILO} = 5/pCMV-luc mit einem N/P-Verhältnis von 4 (n = 6): Messung der Luciferasegenexpression (c). Die Luciferasegenexpression wurde gemessen als Lumineszenz in relativen Lichteinheiten (RLU) während 10 s/mg zellulärem Protein. Die Ergebnisse sind angegeben als Mittelwert ± Standardabweichung. ** bedeutet statistische Signifikanz mit p < 0,01.
Fig. 6 Die in-vivo-Luciferasegenexpression, die mit PEI/pCpG-luc und PEI-g-ILO F_{ILO=5}/pCpG-luc-Teilchen mit einem Verhältnis von N/P = 4 in Lungen von BALB/c-Mäusen nach Aerosolabgabe (n = 5) erhalten wurde. Die Biolumineszenzbilder mit einer Belichtungszeit von 10 min nach 24 h (a). Luciferaseexpressionsmessung in Lungenhomogenisaten von Mäusen mit einer Belichtungszeit von 30 s wurde 24 h nach der Transfektion durchgeführt (b). Die Ergebnisse sind angegeben als vertikaler Punkt mit Median. ** bedeutet statistische Signifikanz mit p < 0,01.

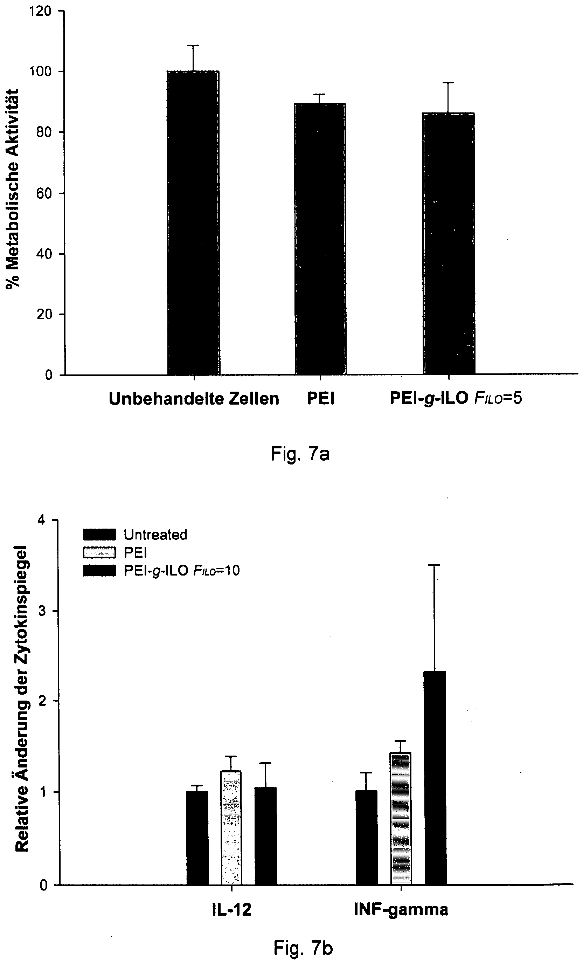

Fig. 7 Die Transfektion von Alveolar-(A549)- und Bronchial-(16HBE14o-), BEAS-2B)-Epithelzellen mit PEI-g-ILO F_{ILO=5}/pCMV-luc-Teilchen mit N/P 4 führte im Vergleich zu PEI/pCMV-luc-Teilchen bzw. im Vergleich zu unbehandelten Zellen nicht zu einem Anstieg der metabolischen Toxizität, was mit einem MTT-Test gemessen wurde (a). Weiterhin wurden nach der Abgabe von PEI-g-ILO F_{ILO=10} an die Lungen von Mäusen im Vergleich zur Abgabe von PEI und unbehandelten Mäusen Interleukin-12 (IL-12) und Interferon-γ (IFN-γ) im Mausserum gemessen. Es zeigte sich kein signifikanter Anstieg dieser Cytokine.

### Beispiel 2

### Dosisabhängige Genvektorzuführung in Lungenzellen

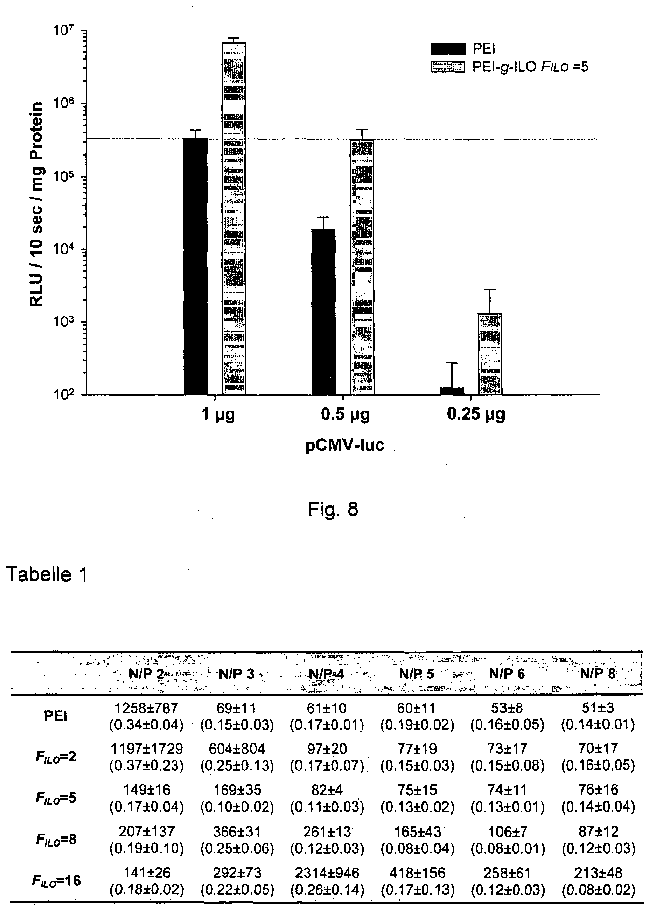

16HBE14o-Zellen wurden mit PEI und PEI-g-ILO-Genvektorteilchen transfiziert, indem die Menge an pCMV-luc von 1 µg auf 0,25 µg verringert wurde (Fig. 8). 24 h nach der Transfektion nahm die Gentransfereffizienz in dosisabhängiger Art und Weise ab. Der höchste Grad an Genexpression wurde gefunden mit 1 µg pCMV-luc. 0,5 µg pCMV-luc komplexiert mit PEI-g-ILO F_{ILO} = 5 führten jedoch zu einer Expression, die gleich war 1 µg pCMV-luc komplexiert mit unmodifiziertem PEI (3,3*10⁵ gegenüber 3,2*10⁵ RLU/10 s/mg Protein).

Um zu zeigen, dass die Gentransfereffizienz in dosisabhängiger Art und Weise abnimmt, wurde ein Transfektionsversuch durchgeführt, bei dem die Menge an Genvektorteilchen reduziert wurde. Hier wurde gezeigt, dass die Reduktion von PEI-g-ILO F_{ILO} = 5 Genvektorteilchen bis auf 50% zu einer gleichen Expression führt im Vergleich zu 100% PEI-Genvektorteilchen. Diese Daten zeigen eindeutig, dass die Menge an pDNA und Genträger reduziert werden kann und der gleiche Expressionsgrad erhalten bleibt. Weiterhin kann sowohl pDNA als auch die trägervermittelte Toxizität und Entzündung reduziert werden.

Die Ergebnisse dieses Beispiels sind auch in Figur 8 dargestellt.

Im Folgenden sind die Literaturstellen angegeben, auf die in der Beschreibung Bezug genommen wird.
1. Gill DR, Davies LA, Pringle IA, Hyde SC. The development of gene therapy for diseases of the lung. Cell Mol Life Sci. 2004 Feb;61(3):355-68.
2. Gurunathan S, Klinman DM, Seder RA. DNA vaccines: immunology, application, and optimization*. Annu Rev Immunol. 2000;18:927-74.
3. Davies L, Hyde, SC and Gill, DR Plasmid inhalation: delivery to the airways; 2005.
4. Rudolph C, Schillinger U, Ortiz A, Plank C, Golas MM, Sander B, et al. Aerosolized nanogram quantities of plasmid DNA mediate highly efficient gene delivery to mouse airway epithelium. Mol Ther. 2005 Sep;12(3):493-501.
5. Canonico AE, Conary JT, Meyrick BO, Brigham KL. Aerosol and intravenous transfection of human alpha 1-antitrypsin gene to lungs of rabbits. Am J Respir Cell Mol Biol. 1994 Jan;10(1):24-9.
6. McLachlan G, Baker A, Tennant P, Gordon C, Vrettou C, Renwick L, et al. Optimizing aerosol gene delivery and expression in the ovine lung. Mol Ther. 2007 Feb; 15(2):348-54.
7. Alton EW, Stern M, Farley R, Jaffe A, Chadwick SL, Phillips J, et al. Cationic lipidmediated CFTR gene transfer to the lungs and nose of patients with cystic fibrosis: a double-blind placebo-controlled trial. Lancet. 1999 Mar 20;353(9157):947-54.
8. Boussif O, Lezoualc'h F, Zanta MA, Mergny MD, Scherman D, Demeneix B, et al. A versatile vector for gene and oligonucleotide transfer into cells in culture and in vivo: polyethylenimine. Proc Natl Acad Sci U S A. 1995 Aug 1;92(16):7297-301.
9. Dunlap DD, Maggi A, Soria MR, Monaco L. Nanoscopic structure of DNA condensed for gene delivery. Nucleic Acids Res. 1997 Aug 1;25(15):3095-101.
10. Kircheis R, Kichler A, Wallner G, Kursa M, Ogris M, Felzmann T, et al. Coupling of cell-binding ligands to polyethylenimine for targeted gene delivery. Gene Ther. 1997 May;4(5):409-18.
11. Chul Cho K, Hoon Jeong J, Jung Chung H, Joe CO, Wan Kim S, Gwan Park T. Folate receptor-mediated intracellular delivery of recombinant caspase-3 for inducing apoptosis. J Control Release. 2005 Nov 2;108(1):121-31.
12. Elfinger M, Maucksch C, Rudolph C. Characterization of lactoferrin as a targeting ligand for nonviral gene delivery to airway epithelial cells. Biomaterials. 2007 Aug;28(23):3448-55.
13. Elfinger M, Geiger J, Hasenpusch G, Uzgun S, Sieverling N, Aneja MK, et al. Targeting of the beta(2)-adrenoceptor increases nonviral gene delivery to pulmonary epithelial cells in vitro and lungs in vivo. J Control Release. 2009 May 5;135(3):234-41.
14. Blessing T, Kursa M, Holzhauser R, Kircheis R, Wagner E. Different strategies for formation of pegylated EGF-conjugated PEI/DNA complexes for targeted gene delivery. Bioconjug Chem. 2001 Jul-Aug;12(4):529-37.
15. Coleman RA, Smith WL, Narumiya S. International Union of Pharmacology classification of prostanoid receptors: properties, distribution, and structure of the receptors and their subtypes. Pharmacol Rev. 1994 Jun;46(2):205-29.
16. Narumiya S, Sugimoto Y, Ushikubi F. Prostanoid receptors: structures, properties, and functions. Physiol Rev. 1999 Oct;79(4):1193-226.
17. Stitham J, Arehart EJ, Gleim SR, Douville KL, Hwa J. Human prostacyclin receptor structure and function from naturally-occurring and synthetic mutations. Prostaglandins Other Lipid Mediat. 2007 Jan;82(1-4):95-108.
18. Clark RB, Knoll BJ, Barber R. Partial agonists and G protein-coupled receptor desensitization. Trends Pharmacol Sci. 1999 Jul;20(7):279-86.
19. Ferguson SS. Evolving concepts in G protein-coupled receptor endocytosis: the role in receptor desensitization and signaling. Pharmacol Rev. 2001 Mar;53(1):1-24.
20. Strauss WL, Edelman JD. Prostanoid therapy for pulmonary arterial hypertension. Clin Chest Med. 2007 Mar;28(1):127-42; ix.
21. Snyder SL, Sobocinski PZ. An improved 2,4,6-trinitrobenzenesulfonic acid method for the determination of amines. Anal Biochem. 1975 Mar;64(1):284-8.
22. Ungaro F, De Rosa G, Miro A, Quaglia F. Spectrophotometric determination of polyethylenimine in the presence of an oligonucleotide for the characterization of controlled release formulations. J Pharm Biomed Anal. 2003 Feb 5;31(1):143-9.
23. Huth S, Lausier J, Gersting SW, Rudolph C, Plank C, Welsch U, et al. Insights into the mechanism of magnetofection using PEI-based magnetofectins for gene transfer. J Gene Med. 2004 Aug;6(8):923-36.
24. Rudolph C, Ortiz A, Schillinger U, Jauernig J, Plank C, Rosenecker J. Methodological optimization of polyethylenimine (PEI)-based gene delivery to the lungs of mice via aerosol application. J Gene Med. 2005 Jan;7(1):59-66.
25. Buckley SM, Howe SJ, Rahim AA, Buning H, Mclntosh J, Wong SP, et al. Luciferin detection after intranasal vector delivery is improved by intranasal rather than intraperitoneal luciferin administration. Hum Gene Ther. 2008 Oct;19(10): 1050-6.
26. Zhang Z, Austin SC, Smyth EM. Glycosylation of the human prostacyclin receptor: role in ligand binding and signal transduction. Mol Pharmacol. 2001 Sep;60(3):480-7.
27. Bley KR, Bhattacharya A, Daniels DV, Gever J, Jahangir A, O'Yang C, et al. RO1138452 and RO3244794: characterization of structurally distinct, potent and selective IP (prostacyclin) receptor antagonists. Br J Pharmacol. 2006 Feb; 147(3):335-45.
28. Clark RD, Jahangir A, Severance D, Salazar R, Chang T, Chang D, et al. Discovery and SAR development of 2-(phenylamino) imidazolines as prostacyclin receptor antagonists [corrected]. Bioorg Med Chem Lett. 2004 Feb 23;14(4):1053-6.
29. Olschewski H, Rose F, Schermuly R, Ghofrani HA, Enke B, Olschewski A, et al. Prostacyclin and its analogues in the treatment of pulmonary hypertension. Pharmacol Ther. 2004 May;102(2):139-53.
30. Skoro-Sajer N, Lang I. Treprostinil for the treatment of pulmonary hypertension. Expert Opin Pharmacother. 2008 Jun;9(8):1415-20.
31. Krug S, Sablotzki A, Hammerschmidt S, Wirtz H, Seyfarth HJ. Inhaled iloprost for the control of pulmonary hypertension. Vasc Health Risk Manag. 2009;5(1):465-74.
32. Ayer LM, Wilson SM, Traves SL, Proud D, Giembycz MA. 4,5-Dihydro-1H-imidazol-2-yl)-[4-(4-isopropoxy-benzyl)-phenyl]-amine (R01138452) is a selective, pseudo-irreversible orthosteric antagonist at the prostacyclin (IP)-receptor expressed by human airway epithelial cells: IP-receptor-mediated inhibition of CXCL9 and CXCL10 release. J Pharmacol Exp Ther. 2008 Feb;324(2):815-26.
33. Boie Y, Rushmore TH, Darmon-Goodwin A, Grygorczyk R, Slipetz DM, Metters KM, et al. Cloning and expression of a cDNA for the human prostanoid IP receptor. J Biol Chem. 1994 Apr 22;269(16):12173-8.
34. Namba T, Oida H, Sugimoto Y, Kakizuka A, Negishi M, Ichikawa A, et al. cDNA cloning of a mouse prostacyclin receptor. Multiple signaling pathways and expression in thymic medulla. J Biol Chem. 1994 Apr 1;269(13):9986-92.
35. Giovanazzi S, Accomazzo MR, Letari O, Oliva D, Nicosia S. Internalization and down-regulation of the prostacyclin receptor in human platelets. Biochem J. 1997 Jul 1;325 (Pt 1):71-7.
36. Smyth EM, Austin SC, Reilly MP, FitzGerald GA. Internalization and sequestration of the human prostacyclin receptor. J Biol Chem. 2000 Oct 13;275(41):32037-45.
37. Huth S, Hoffmann F, von Gersdorff K, Laner A, Reinhardt D, Rosenecker J, et al. Interaction of polyamine gene vectors with RNA leads to the dissociation of plasmid DNA-carrier complexes. J Gene Med. 2006 Dec;8(12):1416-24.
38. Rejman J, Oberle V, Zuhorn IS, Hoekstra D. Size-dependent internalization of particles via the pathways of clathrin- and caveolae-mediated endocytosis. Biochem J. 2004 Jan 1;377(Pt 1):159-69.
39. Hyde SC, Pringle IA, Abdullah S, Lawton AE, Davies LA, Varathalingam A, et al. CpG-free plasmids confer reduced inflammation and sustained pulmonary gene expression. Nat Biotechnol. 2008 May;26(5):549-51.
40. Rudolph C, Muller RH, Rosenecker J. Jet nebulization of PEI/DNA polyplexes: physical stability and in vitro gene delivery efficiency. J Gene Med. 2002 Jan-Feb;4(1):66-74.
41. Gautam A, Densmore CL, Waldrep JC. Pulmonary cytokine responses associated with PEI-DNA aerosol gene therapy. Gene Ther. 2001 Feb;8(3):254-7.

Des Weiteren, betrifft die vorliegende Erfindung die folgenden Gegenstände:
1. Konjugat aus Agens-Komplex und mindestens einem Zielfindungsliganden, wobei der Agens-Komplex ein Agens umhüllt von einem Hüllmaterial umfasst und wobei der Zielfindungsligand ein Prostacyclinanalogon ist.
2. Konjugat nach 1, dadurch gekennzeichnet, dass das Prostacyclinanalogon Iloprost oder Treprostinil ist.
3. Konjugat nach 1 oder 2, dadurch gekennzeichnet, dass das Agens eine Nukleinsäure oder ein Derivat davon, ein Peptid, Polypeptid oder Derivat davon, ein Wirkstoff oder ein Tracer ist.
4. Konjugat nach 3, dadurch gekennzeichnet, dass die Nukleinsäure eine DNA oder RNA ist, deren Mangel oder Defizienz eine Krankheit verursacht oder eine DNA oder RNA ist, die ein Polypeptid kodiert, dessen Mangel oder Defizienz eine Krankheit verursacht oder das immunmodulierend wirkt.
5. Konjugat nach 3, dadurch gekennzeichnet, dass das Agens ein Peptid oder Polypeptid ist, dessen Mangel oder Defizienz eine Krankheit verursacht oder das immunmodulierend wirkt.
6. Konjugat nach einem der vorhergehenden Gegenstände, dadurch gekennzeichnet, dass das Agens ein einen Proteindefekt oder Mangel ausgleichendes Mittel ausgewählt aus Nukleinsäure, Protein, Proteinderivat oder Proteinfragment oder einem in der Lunge wirksamen Arzneimittel oder eine Mischung davon ist.
7. Konjugat nach 3, dadurch gekennzeichnet, dass der Wirkstoff ein entzündungshemmender Wirkstoff oder ein Steroid ist
8. Konjugat nach einem der vorhergehenden Gegenstände, dadurch gekennzeichnet, dass das Agens ein Reportermolekül, insbesondere ein radioaktiver oder fluoreszierender Tracer ist.
9. Konjugat nach einem der vorhergehenden Gegenstände, dadurch gekennzeichnet, dass das Hüllmaterial eine natürlich vorkommende, synthetische oder derivatisierte natürliche Substanz, wie ein Lipid oder ein Polymer oder Oligomer ist.
10. Konjugat nach einem der vorhergehenden Gegenstände, dadurch gekennzeichnet, dass das Hüllmaterial ein kationisches Polymer wie ein biologisch abbaubares stickstoffhaltiges, bevorzugt protonierbares Polymer, insbesondere Polyethylenimin oder bioabbaubares Polyethylenimin, ein kationisches Oligomer, wie Spermin, oder ein kationisches Lipid ist.
11. Konjugat nach einem der vorhergehenden Gegenstände, dadurch gekennzeichnet, dass der Agens-Komplex aus Hüllmaterial und Nukleinsäure zusätzlich pegyliert ist.
12. Konjugat nach einem der vorhergehenden Gegenstände zur Verwendung zur Gentherapie oder zur Verwendung zur Behandlung einer auf einem Proteindefekt oder genetischen Defekt beruhenden pulmonalen Krankheit, insbesondere von zystischer Fibrose.
13. Konjugat nach einem der vorhergehenden Gegenstände, dadurch gekennzeichnet, dass das Verhältnis von kationischem Polymer zu Nukleinsäure, gemessen als molares Verhältnis von Polymer-Stickstoffanteil zu DNA-Phosphatanteil, in einem Bereich von 10:1 bis 1:20 liegt.
14. Konjugat nach einem der vorhergehenden Gegenstände, dadurch gekennzeichnet, dass das von einem Hüllmaterial umhüllte Agens in Form von Nanokapseln, Nanoteilchen oder Liposomen vorliegt.
15. Prostacyclinanalogon zur Verwendung zur Behandlung von Lungenkrankheiten als Zielfindungsstruktur für ein in die Bronchien- und/oder Alveolarepithelzellen zu transportierendes Agens.
16. Therapeutische Zusammensetzung zur Behandlung von Lungenleiden enthaltend ein Konjugat nach einem der 1 bis 15 und übliche pharmazeutische Hilfsstoffe in einer zur Inhalation geeigneten Form.

## Patentansprüche

1. Konjugat aus Agens-Komplex und mindestens einem Zielfindungsliganden, wobei der Agens-Komplex ein Agens umhüllt von einem Hüllmaterial umfasst, wobei der Zielfindungsligand ein Prostacyclinanalogon ist und wobei das Hüllmaterial ein Lipid ist.

2. Konjugat nach Anspruch 1, **dadurch gekennzeichnet, dass** das Prostacyclinanalogon Iloprost oder Treprostinil ist.

3. Konjugat nach Anspruch 1 oder Anspruch 2, **dadurch gekennzeichnet, dass** das Agens eine Nukleinsäure oder ein Derivat davon, ein Peptid, Polypeptid oder Derivat davon, ein Wirkstoff oder ein Tracer ist.

4. Konjugat nach Anspruch 3, **dadurch gekennzeichnet, dass** die Nukleinsäure eine DNA oder RNA ist, deren Mangel oder Defizienz eine Krankheit verursacht oder eine DNA oder RNA ist, die ein Polypeptid kodiert, dessen Mangel oder Defizienz eine Krankheit verursacht oder das immunmodulierend wirkt.

5. Konjugat nach Anspruch 3, **dadurch gekennzeichnet, dass** das Agens ein Peptid oder Polypeptid ist, dessen Mangel oder Defizienz eine Krankheit verursacht oder das immunmodulierend wirkt.

6. Konjugat nach einem der vorhergehenden Ansprüche, **dadurch gekennzeichnet, dass** das Agens ein einen Proteindefekt oder Mangel ausgleichendes Mittel ausgewählt aus Nukleinsäure, Protein, Proteinderivat oder Proteinfragment oder einem in der Lunge wirksamen Arzneimittel oder eine Mischung davon ist.

7. Konjugat nach Anspruch 3, **dadurch gekennzeichnet, dass** der Wirkstoff ein entzündungshemmender Wirkstoff oder ein Steroid ist.

8. Konjugat nach einem der vorhergehenden Ansprüche, **dadurch gekennzeichnet, dass** das Agens ein Reportermolekül, insbesondere ein radioaktiver oder fluoreszierender Tracer ist.

9. Konjugat nach einem der vorhergehenden Ansprüche, **dadurch gekennzeichnet, dass** das Hüllmaterial ein natürlich vorkommendes, synthetisches oder derivatisiertes Lipid ist.

10. Konjugat nach einem der vorhergehenden Ansprüche, **dadurch gekennzeichnet, dass** das Hüllmaterial ein kationisches Lipid ist.

11. Konjugat nach einem der vorhergehenden Ansprüche, **dadurch gekennzeichnet, dass** der Agens-Komplex aus Hüllmaterial und Nukleinsäure zusätzlich pegyliert ist.

12. Konjugat nach einem der vorhergehenden Ansprüche zur Verwendung zur Gentherapie oder zur Verwendung zur Behandlung einer auf einem Proteindefekt oder genetischen Defekt beruhenden pulmonalen Krankheit, insbesondere von zystischer Fibrose.

13. Konjugat nach einem der Ansprüche 1 bis 11 oder Konjugat zur Verwendung nach Anspruch 12, **dadurch gekennzeichnet, dass** das Verhältnis von Hüllmaterial zu Agens, bezogen auf Gewicht, in einem Bereich von 10:1 bis 1:4 liegt.

14. Konjugat nach einem der Ansprüche 1 bis 11 und 13 oder Konjugat zur Verwendung nach Anspruch 12 oder 13, **dadurch gekennzeichnet, dass** das von einem Hüllmaterial umhüllte Agens in Form von Liposomen vorliegt.

15. Konjugat nach einem der Ansprüche 1 bis 11, 13 und 14 zur Verwendung zur Behandlung von Lungenkrankheiten, wobei das Prostacyclinanalogon als Zielfindungsstruktur den Transfer des Agens in Bronchien- und/oder Alveolarepithelzellen ermöglicht.

16. Therapeutische Zusammensetzung zur Behandlung von Lungenleiden enthaltend ein Konjugat nach einem der Ansprüche 1, 11, 13 und 14.
